(19) Europäisches Patentamt
European Patent Office
Office européen des brevets

(11) Publication number: **0 240 256 B1**

(12) **EUROPEAN PATENT SPECIFICATION**

(45) Date of publication of patent specification: **29.04.92**

(51) Int. Cl.5: **C07C 59/90**, C07C 62/38, C07C 233/46, C07C 235/32, C07C 235/70, C07C 205/27, C07C 205/49

(21) Application number: **87302643.9**

(22) Date of filing: **26.03.87**

(54) **Compounds useful in treating sickle cell anemia.**

(30) Priority: **04.04.86 US 848078**
**04.04.86 US 848195**
**04.04.86 US 848080**
**04.04.86 US 848079**

(43) Date of publication of application:
**07.10.87 Bulletin 87/41**

(45) Publication of the grant of the patent:
**29.04.92 Bulletin 92/18**

(84) Designated Contracting States:
**CH DE FR GB IT LI NL**

(56) References cited:
**CH-A- 484 043**
**DE-A- 1 810 053**
**FR-A- 1 525 941**

**E.J. CRAGOE, Jr. et al.: "Diuretics: Chemistry, Pharmacology and Medicine", 1983, John Wiley and Sons, New York, US**

(73) Proprietor: **MERCK & CO. INC.**
**126, East Lincoln Avenue P.O. Box 2000**
**Rahway New Jersey 07065-0900(US)**

Proprietor: **UNIVERSITY OF PITTSBURGH**
**801 Cathedral of Learning**
**Pittsburgh, Pennsylvania 15260(US)**

(72) Inventor: **Abraham, Donald J.**
**3624 Cal Ken Drive**
**Murrysville Pennsylvania 15668(US)**
Inventor: **Cragoe, Edward J., Jr.**
**2211 Oak Terrace Drive**
**Lansdale Pennsylvania 19446(US)**
Inventor: **Woltersdorf, Otto W., Jr.**
**200 Dorset Way**
**Chalfont Pennsylvania 18914(US)**

(74) Representative: **Hesketh, Alan, Dr.**
**European Patent Department Merck & Co., Inc. Terlings Park Eastwick Road**
**Harlow Essex, CM20 2OR(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(-/2.18/2.0)

## Description

U.S. Patent 3,478,085 and 3,255,241 and British Published application 998,835 disclose general structures which include the compounds claimed in the present invention. None of these references, however, specifically disclose the claimed compounds. E. J. Cragoe, Jr., Ed., Diuretics: Chemistry, Pharmacology and Medicine, John Wiley and Sons, New York 1983, discloses two analogues of the claimed compounds on page 213.

### BACKGROUND OF THE INVENTION

This invention relates to compounds useful in treating sickle cell anemia and, more specifically, it relates to a method of resisting sickling of sickle hemoglobin in a sickle cell anemia patient.

Sickle cell anemia is a hereditary blood disease which primarily afflicts people of African, Mediterranean and Mideastern origin or their descendants. The anemia results from the physical aggregation of a mutant hemoglobin protein constituent, hemoglobin S, in red blood cells. This aggregation results in a distortion in shape of deoxygenated red blood cells and causes impairment of flow of the blood through the capillaries (sickle cell "crises"). As the principal function of hemoglobin is to transport oxygen from the lungs to body tissues, efficient flow of oxygen throughout the body's tissues is impeded by the anemia due to a lower number of red blood cells. Sickle cell anemia also may have an indirect effect on the heart, lungs, kidneys, spleen, bones and brain. Sickle cell anemia crises can be extremely painful, can result in infections such as pneumonia, can result in skin ulceration, can contribute to strokes and seizures in the afflicted individuals and can also result in the development of chronic bone infections.

In general, the result of the differences between cells containing hemoglobin A, the normal hemoglobin, and hemoglobin S, the sickle cell hemoglobin, is that in the deoxygenated state the former cell is generally flexible and biconcave discoid in shape, while the latter is more rigid and crescent shaped and typically has pointed ends. This rigidity and distortion in shape causes the cells to be lodged in the capillary. Hemoglobin molecules contain two beta polypeptide chains and two alpha polypeptide chains. In the sickle cell hemoglobin, a mutation is present in the beta chains. More specifically, the sixth amino acid of each beta chain is changed from glutamic acid to valine. As a result of this mutation, hemoglobin S upon deoxygenation polymerizes and causes the cell to assume the elongated, sickle-like configuration. As the sickle cells have a much shorter life span than normal red cells, the body depletes the sickled cells more quickly thereby creating an anemic condition.

Electrophoresis is one of the well established laboratory tests employed in diagnosing sickle cell anemia. Electrophoresis tests determine whether an individual has sickle cell anemia (homozygous) or merely the sickle cell trait (heterozygous). The latter refers to an individual not having the disease but having the capability of transmitting the disease to offspring if mated to another heterozygote.

One major assay for evaluating antisickling agents involves measuring their effect on increasing the concentration at which sickle hemoglobin forms a gel. This is called the solubility or $C_{sat}$ assay.

Another well established laboratory test employed for determining the potential effectiveness of an antisickling agent is by determining the oxygen-disassociation curve. When a graph is plotted of the percentage saturation of hemoglobin with oxygen (ordinate) against the partial pressure of oxygen, sometimes called the oxygen tension (abscissa) a characteristic sigmoid curve is obtained. With respect to the curve obtained with whole blood from normal adults, that obtained with whole blood from sickle-cell anemia sufferers is displaced to the right with a loss of sigmoidicity. That is to say, the hemoglobin in the sickle-cell erythrocytes appears to have a reduced oxygen affinity compared with that in the normal erythrocytes, a higher oxygen tension being required to produce a given percentage saturation. (With whole blood from individuals having the sickle-cell trait the curve is not significantly displaced from the normal).

The compounds of this invention are effective in both increasing sickle hemoglobin solubility toward more normal values ($C_{sat}$ assay) and in left-shifting (normalizing) the oxygen disassociation curve.

Treatment for the various complications which have resulted from sickle cell anemia are known and should be distinguished from prophylactic treatment which is unknown and would eliminate the occurrence of the complications and adverse symptoms. Currently, symptomatic treatment is available. For example, one can treat the symptoms by using analgesics for pain, or antibiotics for infection, but these approaches do not arrest the underlying sickling phenomena.

There remains, therefore, a very real and substantial need for a treatment method which minimizes the adverse consequences of sickle cell anemia by directly inhibiting the underlying cause of sickle cell crises.

The present invention has met the above-described need by providing a method which preferably involves administering to a person a therapeutically effective dosage of a compound of this invention. This dosage is administered by the compound being reacted extracorporeally with the patient's own blood or the agent may be given orally. In the former approach the agent is preferably administered to stored blood samples taken from patients and then the blood is readministered.

It is an object of the present invention to provide a method of treating a sickle cell anemia patient's blood so as to reduce undesired sickle cell crises.

It is another object of the present invention to provide an effective means for resisting undesired sickling of hemoglobin in sickle cell anemia patients.

## DESCRIPTION OF THE INVENTION

The instant invention is directed to compounds of structural formula (I) and pharmaceutically acceptable salts thereof:

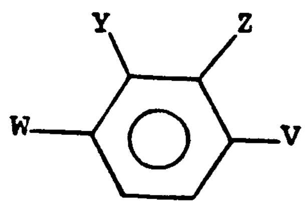

(I)

wherein V is selected from:

(a)

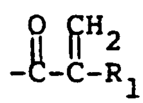

in which

$R_1$ is ethyl, propyl or isopropyl;

(b)

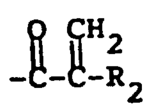

in which

$R_2$ is H or lower alkyl, lower alkyl is preferably $C_{1-4}$;

(c)

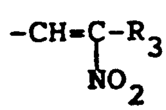

in which

$R_3$ is lower alkyl, preferably $C_{1-4}$ alkyl;

(d)

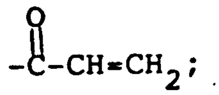

W is selected from:

(a)

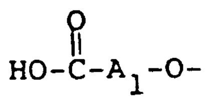

in which

$A_1$ is $(CH_2)n_1$, $-C(CH_2)_3$ and

$n_1 = 2,4$, or 5;

(b)

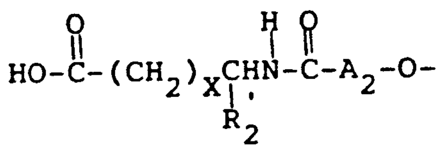

in which

$R_2'$ is H, lower alkyl, benzyl, $CH_2OH$;

preferably H or lower alkyl, most preferably H, lower alkyl is preferably $C_{1-4}$;

$A_2$ is $(CH_2)n_2$ $-C(CH_3)_2$, $-C(CH_2)_3$- and $n_2$ = 1 to 5;

X is 0 or 1;

(c)

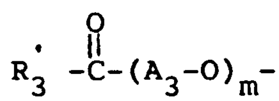

in which

$R_3'$ is HO- or

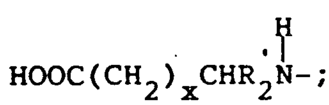

$A_3$ is $-(CH_2)n_3$ or $-C(CH_2)_3$,- and

$n_3$ = 1 to 5;

m is 0 or 1;

(d)

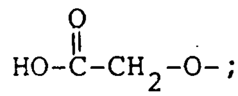

and Y is H, Cl or $OCH_3$;
Z is H or Cl;
provided that
(i) when W is

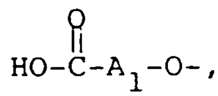

V is

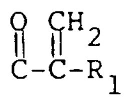

and Y and Z are Cl;
(ii) when W is

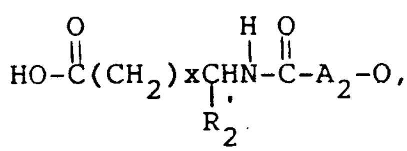

V is

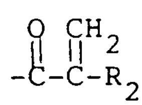

Y and Z are Cl, with the proviso that when $R_2$ is $C_2H_5$, $R_2'$ is H and X is O,

$n_2$ is 2 to 5; this proviso applies only to the compounds and not to the pharmaceutical compositions and methods of treatment described below; provided also that when $R_2$ is isopropyl , $R_2'$ is $CH_3$ and X is O , n is 2 to 5 ;
(iii) when W is

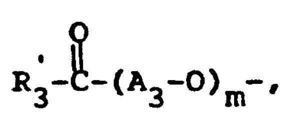

V is

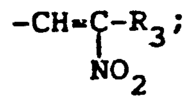

with the proviso that when $R_3'$ is OH, Y is H or Cl and m is 1, $n_3$ = 4 or 5; this proviso applies only to the compounds and not to the pharmaceutical compositions and methods of treatment described below;

(iv) when W is

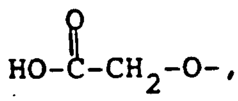

V is

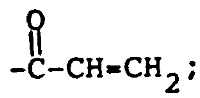

and Y and Z are Cl.

The present invention is also directed to an antisickling pharmaceutical carrier and an antisickling agent of the formula (I) and pharmaceutically acceptable salts thereof. The present invention is also directed to an antisickling pharmaceutical carrier and an antisickling agent of the formula (II) and pharmaceutically acceptable salts thereof:

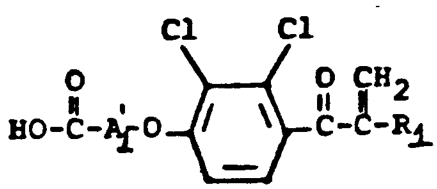

(II)

wherein

$A_1'$ is $-(CH_2)_3-$ or

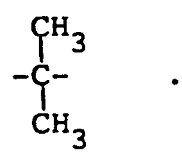

The compounds of the above formulas as defined, may be used in medicine in the palliation of haemoglobinopathies and in particular for alleviating the symptoms of sickle-cell anemia and mitigating the sufferings of those having the condition. The compounds may be used both on a regular maintenance basis and for the relief of acute crisis states.

The compounds may be administered to the human recipient by a route selected from oral, parenteral (including subcutaneous, intradermal, intramuscular and intravenous) rectal and extracorporeally. The size of an effective palliative dose of a compound will depend upon a number of factors including the identity of the recipient, the type of haemoglobinopathy involved, the severity of the condition to be treated and the route of administration and will ultimately be at the discretion of the attendant physician.

One embodiment of the present invention is the compounds of structure III and their pharmaceutical composition and use as antisickling agents.

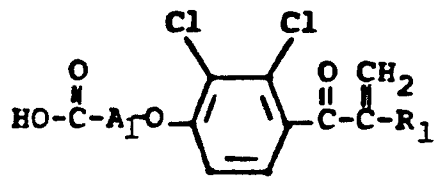

(III)

Exemplifying this embodiment are the following compounds:

3-[2,3-dichloro-4-(2-methylenebutyryl)phenoxy]propionic acid.

5-[2,3-dichloro-4-(2-methylenebutyryl)phenoxy]valeric acid.

6-[2,3-dichloro-4-(2-methylenebutyryl)phenoxy]hexanoic acid.

1-[2,3-dichloro-4-(2-methylenebutyryl)phenoxy]cyclobutane-1-carboxylic acid.

1-[2,3-dichloro-4-(2-methylenevaleryl)phenoxy]cyclobutane-1-carboxylic acid.

A second embodiment of the present invention is the compounds of structure IV and their pharmaceutical composition and use as antisickling agents.

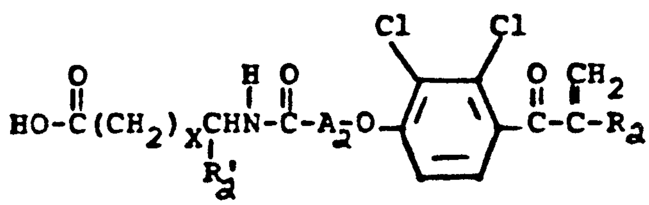

(IV)

Exemplifying this embodiment are the following compounds:

N-[[2,3-dichloro-4-(2-methylenebutyryl)phenoxy]acetyl]-S(+)-alanine.

N-[[2,3-dichloro-4-(2-methylenebutyryl)phenoxy]acetyl]-S(+)-serine.

N-[1-[2,3-dichloro-4-(2-methylenebutyryl)phenoxy]cyclobutane-1-carbonyl]glycine.

N-{1-[2,3-dichloro-4-(2-methylenevaleryl)phenoxy]cyclobutane-1-carbonyl}glycine.

N-{[2,3-dichloro-4-(2-methylenebutyryl)phenoxy]acetyl}-S(-)-phenylalanine.

N-{4-[2,3-dichloro-4-(2-methylenebutyryl)phenoxy]butyryl}glycine.

N-{3-[2,3-dichloro-4-(2-methylenebutyryl)phenoxy]propionyl}glycine.

N-{1-[2,3-dichloro-4-(2-methylenebutyryl)phenoxy]cyclobutane-1-carbonyl)-3-aminopropionic acid.

N-[(2,3-dichloro-4-acryloylphenoxy)acetyl]glycine.

N-{1-[2,3-dichloro-4-(2-methylenebutyryl)phenoxy cyclobutane-1-carbonyl)-S(+)-alanine.

N-{[2,3-dichloro-4-(2-methylenebutyryl)phenoxy]acetyl}S(+)-leucine.

A third embodiment of the present invention is the compounds of structure (V) and their pharmaceutical composition and use as antisickling agents.

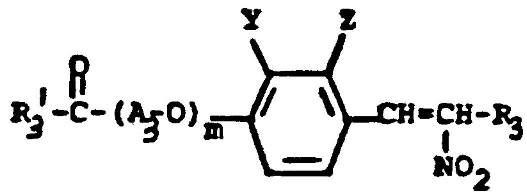

(V)

Exemplifying this embodiment are the following compounds:

| $R_3'$ | $A_3$ | m | $R_3$ | Y | Z |
|---|---|---|---|---|---|
| 1. OH | $(CH_2)_4$ | 0 | $C_2H_5$ | Cl | Cl |
| 2. OH | $(CH_2)_5$ | 0 | $C_2H_5$ | Cl | Cl |
| 3. OH | $-C(CH_2)_3$ | 0 | $C_2H_5$ | Cl | Cl |
| 4. OH | $-C(CH_2)_3$ | 0 | $CH_2CH_2CH_3$ | Cl | Cl |
| 5. $HOOCCH_2NH$ | $(CH_2)_3$ | 0 | $C_2H_5$ | Cl | Cl |
| 6. S(+)-$HOOCCH_2NH$ | $(CH_2)_3$ | 0 | $C_2H_5$ | Cl | Cl |
| 7. S(+) $HOOCCH[CH(CH_3)_2]NH$ | $(CH_2)_3$ | 0 | $C_2H_5$ | Cl | Cl |
| 8. S(+)-$HOOCCH(CH_2OH)NH$ | $(CH_2)_3$ | 0 | $C_2H_5$ | Cl | Cl |
| 9. S(-)-$HOOCCH(CH_2C_6H_5)NH$ | $(CH_2)_3$ | 0 | $C_2H_5$ | Cl | Cl |
| 10. $HOOCCH_2NH$- | $(CH_2)_3$ | 0 | $CH_2CH_2CH_3$ | Cl | Cl |
| 11. $HOOCCH_2NH$ | $-C(CH_2)_3$ | 0 | $C_2H_5$ | Cl | Cl |
| 12. $HOOCCH_2NH$ | $(CH_2)_2$ | 0 | $C_2H_5$ | Cl | Cl |
| 13. $HOOCCH_2NH$ | $(CH_2)_4$ | 0 | $C_2H_5$ | Cl | Cl |
| 14. $HOOCCH_2NH$ | $(CH_2)_3$ | 0 | $C_2H_5$ | Cl | Cl |
| 15. $HOOCCH_2CH_2NH$ | $-C(CH_2)_3$ | 0 | $C_2H_5$ | Cl | Cl |
| 16. OH | $CH_2$ | 1 | $CH_3$ | $OCH_3$ | H |
| 17. OH | - | 0 | | H | Cl |

A fourth embodiment of the present invention is the compound of structure (VI) and its pharmaceutical composition and use as an antisickling agent.

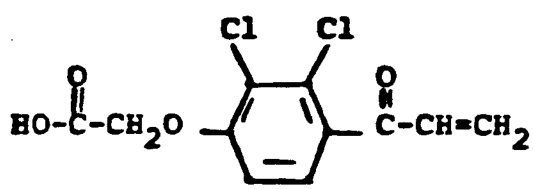

(VI)

A fifth embodiment of the present invention is a pharmaceutical composition containing an anti-sickling agent useful for treating sickle cell anemia wherein the anti-sickling agent is a compound of formula

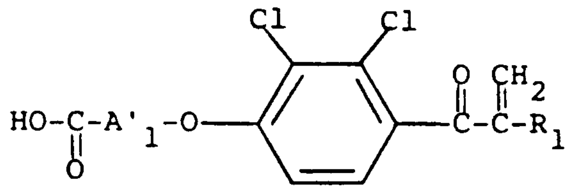

wherein

$A'_1$ is $-(CH_2)_3-$ or

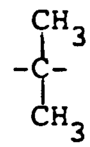

The compounds of structure III are prepared as follows:

(VII)

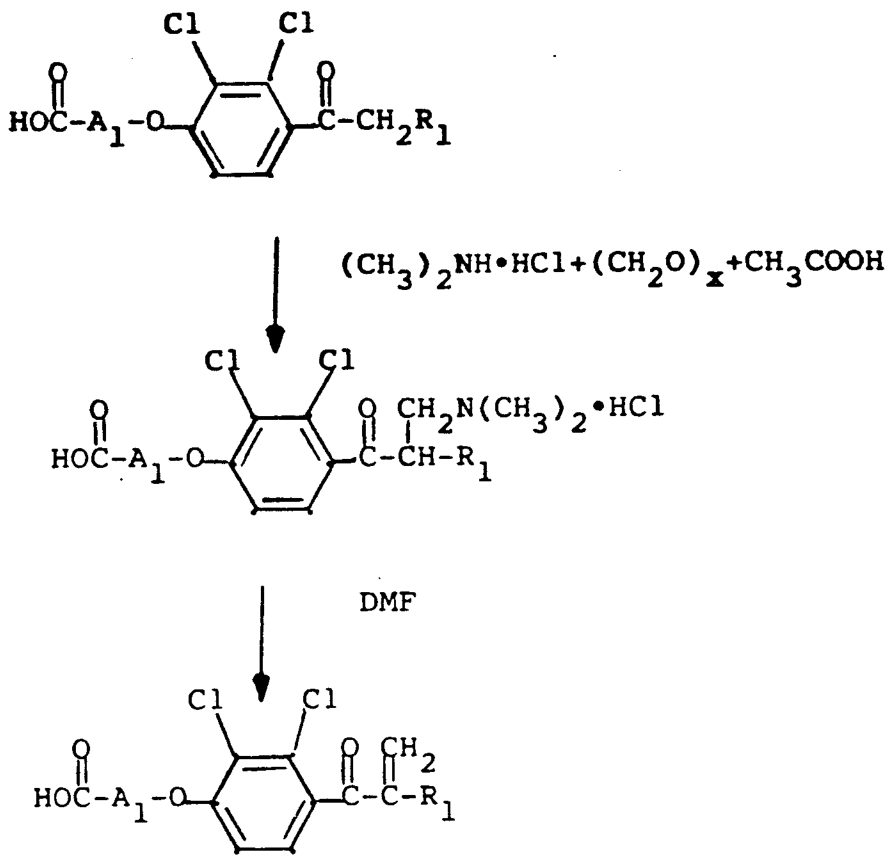


(VIII)

(III)

A compound of Formula VII is reacted under the conditions of the Mannich reaction with dimethylamine hydrochloride, paraformaldehyde and a catalytic amount of acetic acid to form a compound of Formula VIII. The reaction is generally conducted without a solvent at temperatures in the range of 100 ° C for periods of 5 to 12 hours.

It is generally not convenient to isolate the compounds of Formula VIII but to convert them directly to the desired products of Formula III by heating with a solvent, such as N,N-dimethylformamide (DMF) or 1-methyl-2-pyrrolidinone. The reaction is generally complete when heating at 75-110 ° C is carried out for 5-12 hours.

The intermediate compounds of Formula VIIa are prepared as follows for compounds where $A_1 = A_0 = -(CH_2)_3-$, $-(CH_2)_4-$, $-(CH_2)_5-$, or $-C(CH_2)_3$,

(X) (IX)

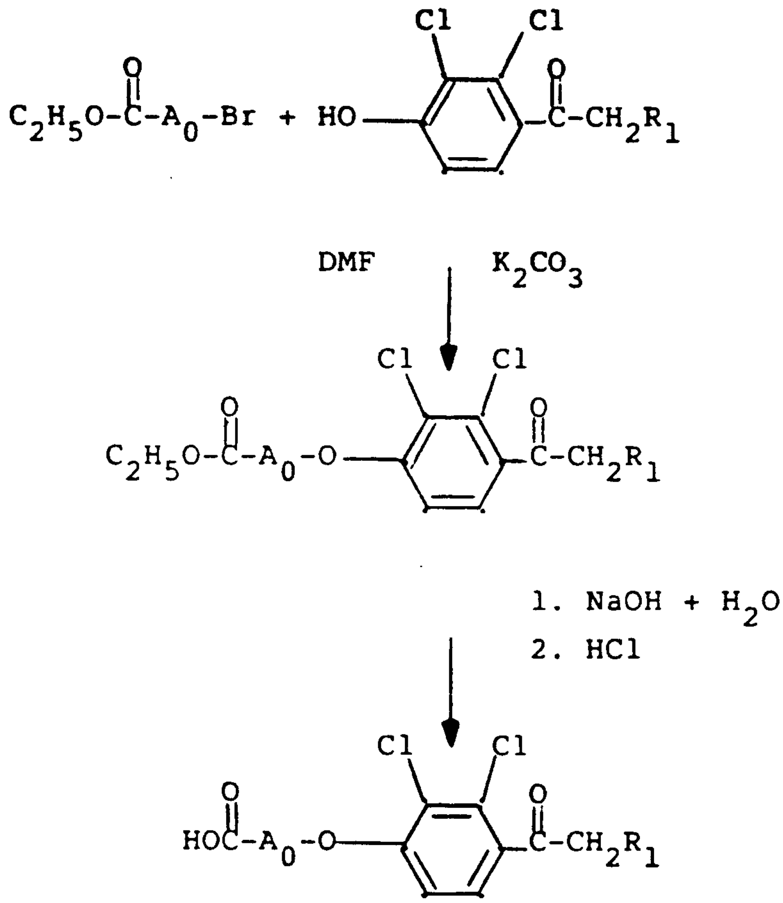


(XI)

(VIIa)

A phenol of Formula IX is reacted with a bromo ester of Formula X to produce an ester of Formula XI. The reaction is conducted in any one of several solvents, such as N,N-dimethylformamide (DMF) or 1-methyl-2-pyrrolidinone in the presence of a base, such as potassium carbonate or sodium carbonate. The reaction temperature is generally of 25 to 100 ° C, preferably at 55-60 ° C for a period of one to 48 hours, preferably 10-20 hours.

The esters of Formula XI are converted to the corresponding carboxylic acids of Formula VIIa by saponification with aqueous sodium hydroxide followed by acidification. Saponification is conveniently conducted in the presence of the N,N-dimethylformamide solvent used for making the ester. Saponification is generally complete within one to five hours when the reaction temperature is in the range of 75-100 ° C.

When $A_1$ is $-(CH_2)_2-$, the compounds of Formula VIIb are prepared as follows:

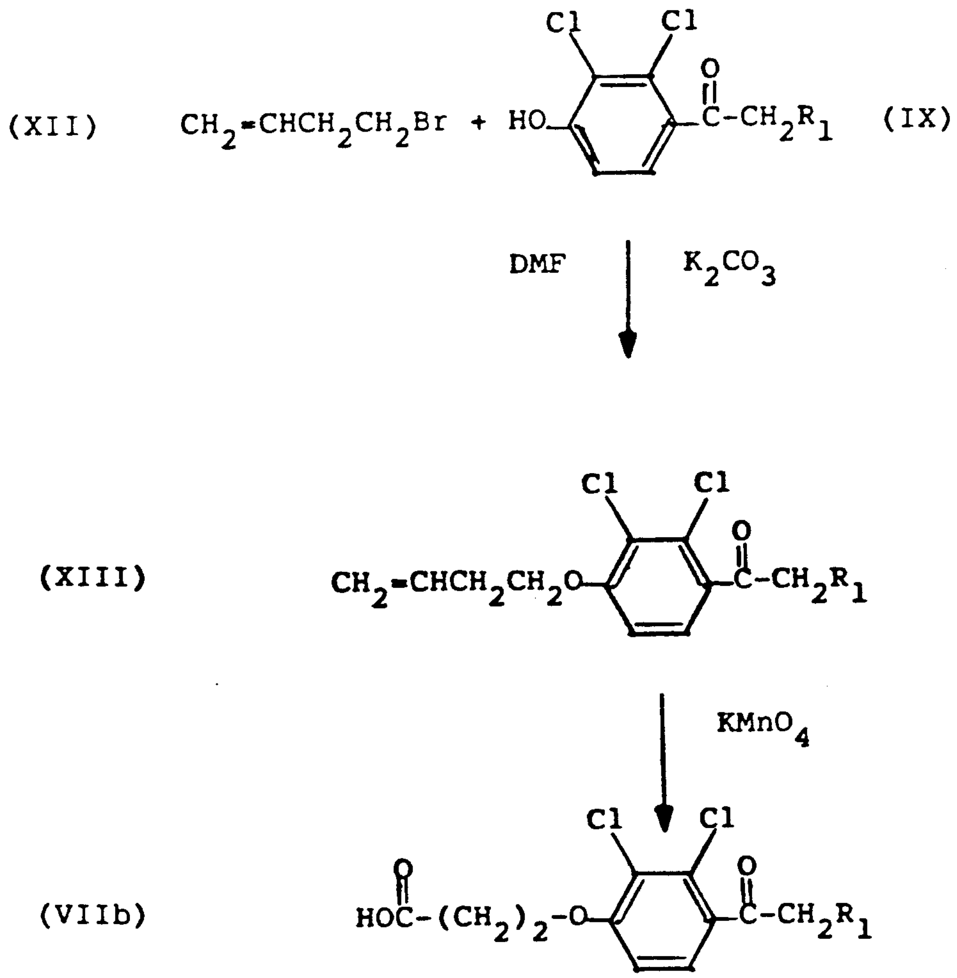


(XII) (IX) (XIII) (VIIb)

Reaction of a phenol of Formula IX with 4-bromo-1-butene (XII) in a solvent, such as N,N-dimethylformamide or 1-methyl-2-pyrrolidinone and a base, such as potassium carbonate or sodium carbonate gives ethers of Formula XIII.

Compounds of Formula XIII are converted to compounds of Formula VIIb by oxidation. The reaction is conducted by stirring a two-phase mixture of a compound of Formula XIII in methylene chloride with an aqueous solution of potassium permanganate at a temperature of 0 to 10° C for 30 minutes to 3 hours. A detergent, e.g., benzyltriethylammonium chloride is used to promote the two-phase reaction. The product is isolated by extracting the methylene chloride layer with aqueous sodium hydroxide followed by acidification of the aqueous extract with hydrochloric acid.

When $A_1$ is $-C(CH_3)_2-$ the compounds of Formula VIIc are prepared as follows:

(IX)

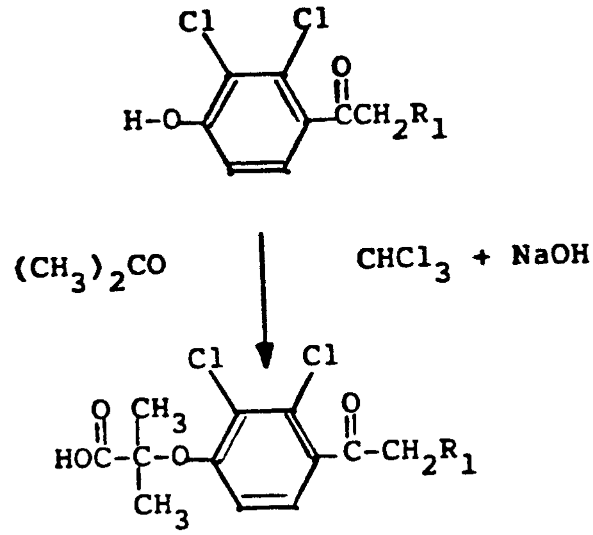


(VIIc)

The reaction of a phenol of Formula IX with acetone and chloroform in the presence of sodium hydroxide produces compounds of Formula VIIc. The reaction is conducted by adding chloroform dropwise to a mixture of a compound of Formula IX in acetone followed by refluxing the mixture for a period of 1 to 6 hours. The product is isolated by evaporating the acetone, treating the residue with water, acidifying the mixture, extracting with ether, drying the ether and removing the solvent. The product is conveniently purified by chromatography.

The salts of compounds of Formula III are also a part of the instant invention. These compounds are prepared by reacting the acids of Formula III with an appropriate base, for example, alkali metal or alkaline earth bicarbonate, carbonate or alkoxide, an amine, ammonia, an organic quaternary ammonium hydroxide, guanidine and the like.

The reaction is generally conducted in water when alkali metal bicarbonates or carbonates are used, but when alkoxides and the organic bases are used, the reaction may be conducted in an organic solvent, such as ether, ethanol, N,N-dimethylformamide and the like.

The preferred salts are the pharmaceutically acceptable salts such as sodium, potassium, ammonium and the like.

The compounds of Formula IV are prepared as follows:

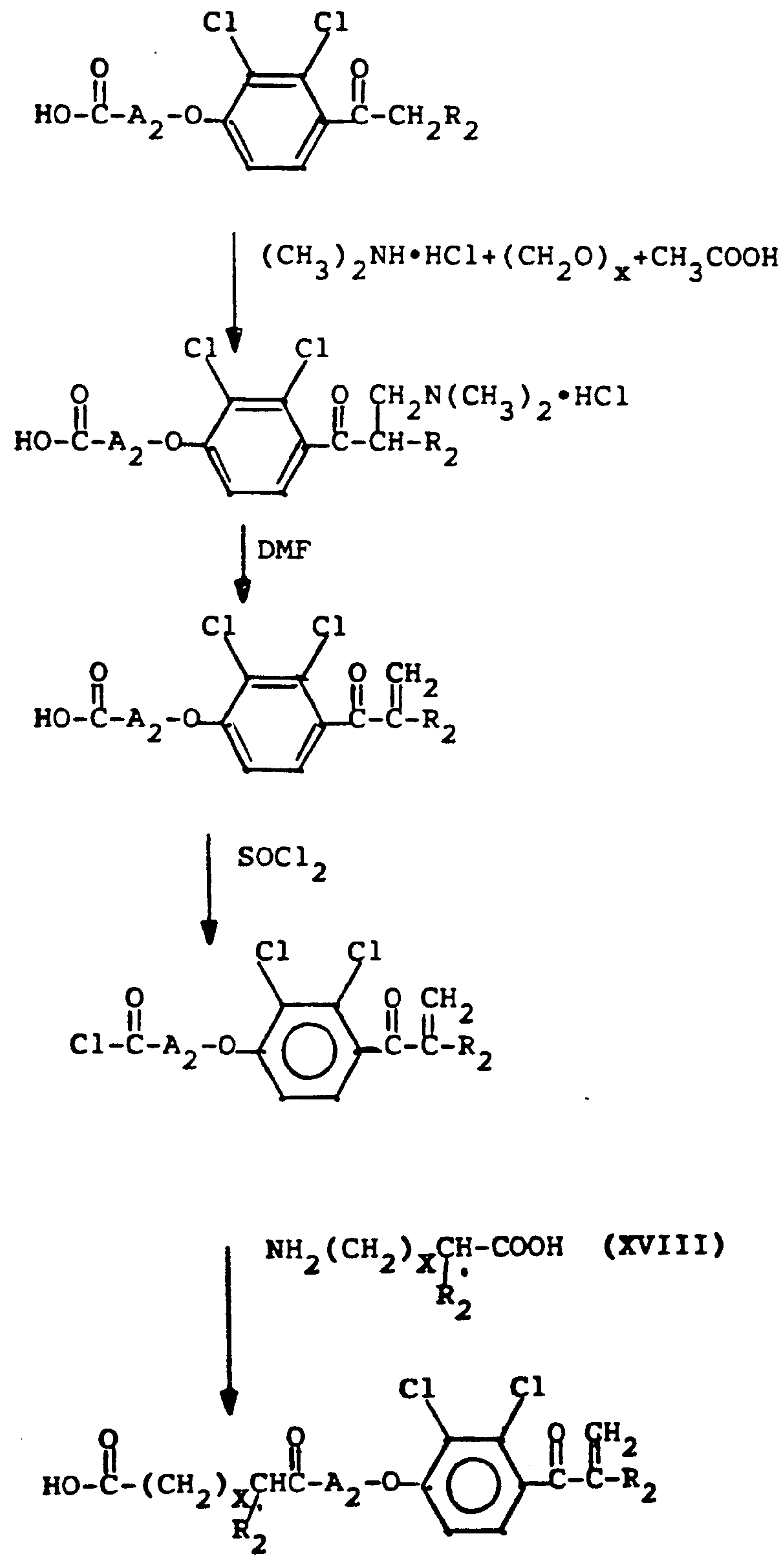


(XIV)

(XV)

(XVI)

(XVII)

(IV)

A compound of Formula XIV is reacted under the conditions of the Mannich reaction with dimethylamine hydrochloride, paraformaldehyde and a catalytic amount of acetic acid to form a compound of Formula XV. The reaction is generally conducted without a solvent at temperatures in the range of 100 °C for periods of 5 to 12 hours.

It is generally not convenient to isolate, the compounds of Formula XV but to convert them directly to the desired products of Formula XVI by heating with a solvent, such as N,N-dimethylformamide (DMF) or 1-methyl-2-pyrrolidinone. The reaction is generally complete when heating at 75-110 °C is carried out for 5-12 hours.

A compound of Formula XVII is prepared by treating a compound of Formula XVI with thionyl chloride or similar reagent in an inert solvent such as benzene, toluene, methylene chloride, carbon tetrachloride and the like. The reaction is conducted at temperatures of 40 °C to that of the boiling point of the solvent, preferably, if possible, in the range of 60-80 °C for periods of time of 30 minutes to 6 hours. During this time the hydrogen chloride generated during the reaction is evolved.

The reaction of a compound of Formula XVII with two moles of a compound of an amino acid of Formula XVIII gives a compound of Formula IV.

It should be noted that when $R'_2$ of Formula XVIII is other than H, the product of Formula IV can well be either racemic or the R- or S-enantiomer depending on the structure of Formula XVIII used in the reaction.

The intermediate compounds of Formula XIVa are prepared as follows for compounds where $A_2 = (CH_2)n'_2$ or $-C(CH_2)_3$, and where $n'_2 = 1, 3, 4$ or 5.

(XX)

(XIX)

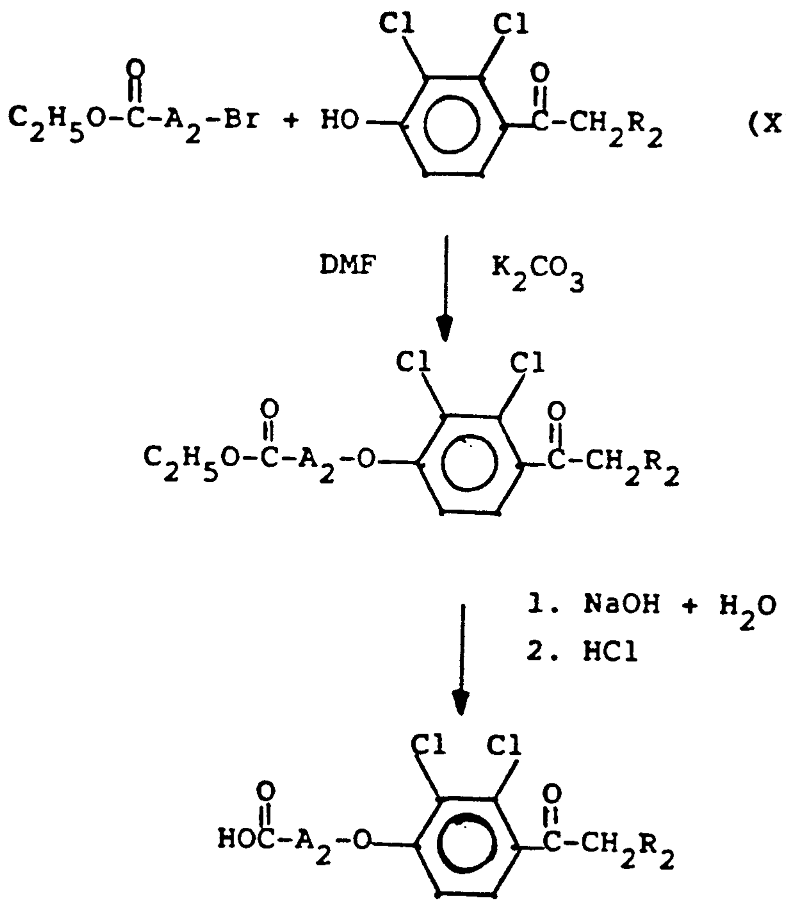


(XXI)

(XIVa)

A phenol of Formula XIX is reacted with a bromo ester of Formula XX to produce an ester of Formula XXI. The reaction is conducted in any one of several solvents, such as N,N-dimethylformamide (DMF) or 1-methyl-2-pyrrolidinone in the presence of a base, such as potassium carbonate or sodium carbonate. The reaction temperature is generally of 25 to 100 °C preferably at 55-60 °C for a period of one to 48 hours, preferably 10-20 hours.

The esters of Formula XXI are converted to the corresponding carboxylic acids of Formula XIVa by saponification with aqueous sodium hydroxide followed by acidification. Saponification is conveniently conducted in the presence of the N,N-dimethylformamide solvent used for making the ester. Saponification is generally complete within one to five hours when the reaction temperature is in the range of 75-100 °C.

When $A_2$ is $-(CH_2)_2-$, the compounds of Formula XIVb are prepared as follows:

(XII)

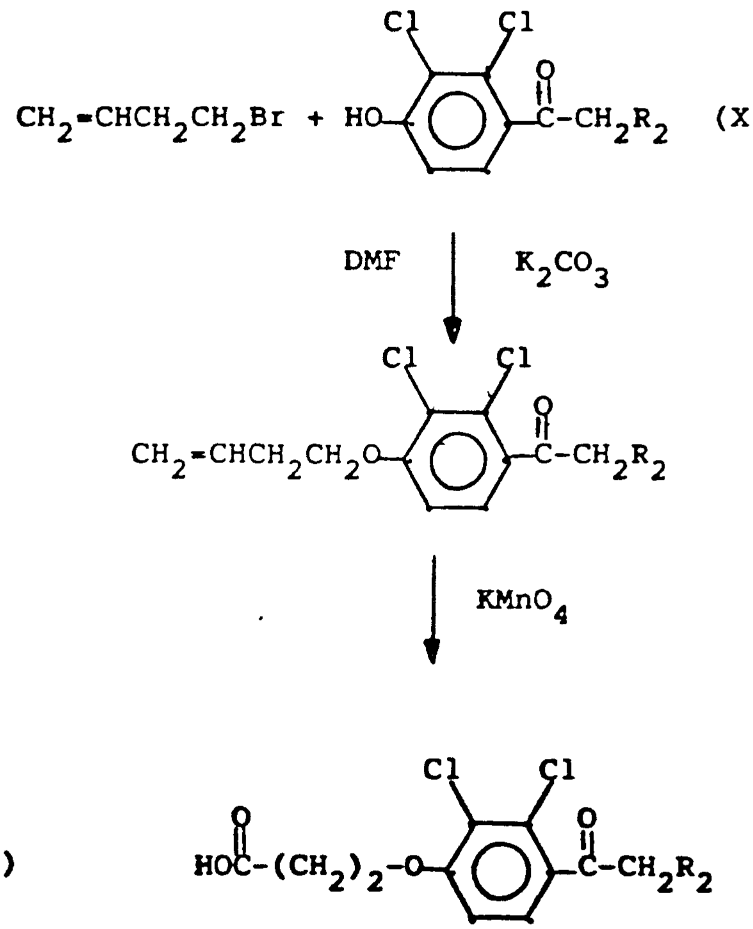


(XIX)

(XXII)

(XIVb)

Reaction of a phenol of Formula XIX with 4-bromo-1-butene (XII) in a solvent, such as N,N-dimethylformamide or 1-methyl-2-pyrrolidinone and a base, such as potassium carbonate or sodium carbonate gives ethers of Formula XXII.

Compounds of Formula XXII are converted to compounds of Formula XIVb by oxidation. The reaction is conducted by stirring a two-phase mixture of a compound of Formula XXII in methylene chloride with an aqueous solution of potassium permanganate at a temperature of 0 to 10 °C for 30 minutes to 3 hours. A detergent, e.g., benzyltriethylammonium chloride is used to promote the two-phase reaction. The product is isolated by extracting the methylene chloride layer with aqueous sodium hydroxide followed by acidification of the aqueous extract with hydrochloric acid.

When $A_2$ is $-C(CH_3)_2-$ the compounds of Formula XIVC are prepared as follows:

(XIX)

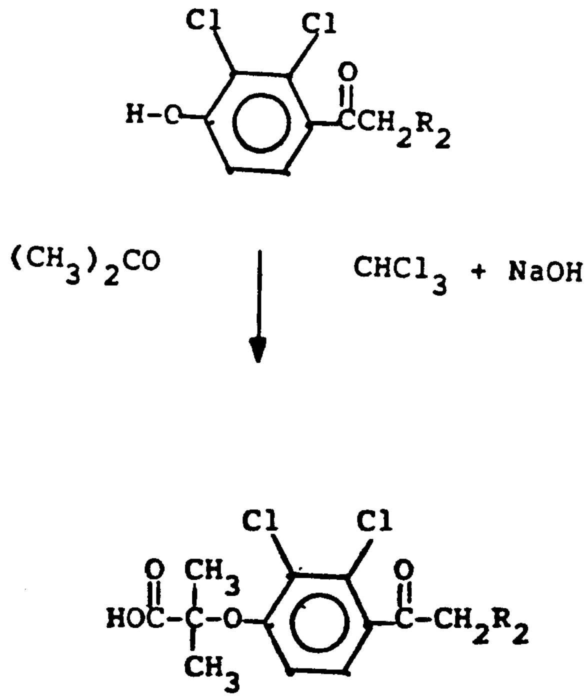


(XIVc)

The reaction of a phenol of Formula XIX with acetone and chloroform in the presence of sodium hydroxide produces compounds of Formula XIVc upon acidification. The reaction is conducted by adding chloroform dropwise to a mixture of a compound of Formula XIX in acetone followed by refluxing the mixture for a period of 1 to 6 hours. The product is isolated by evaporating the acetone, treating the residue with water, acidifying the mixture, extracting with ether, drying the ether and removing the solvent. The product is conveniently purified by chromatography.

Intermediate compounds of Formula XIVd where R = H are prepared as follows:

(XXIII)
(XXIV)
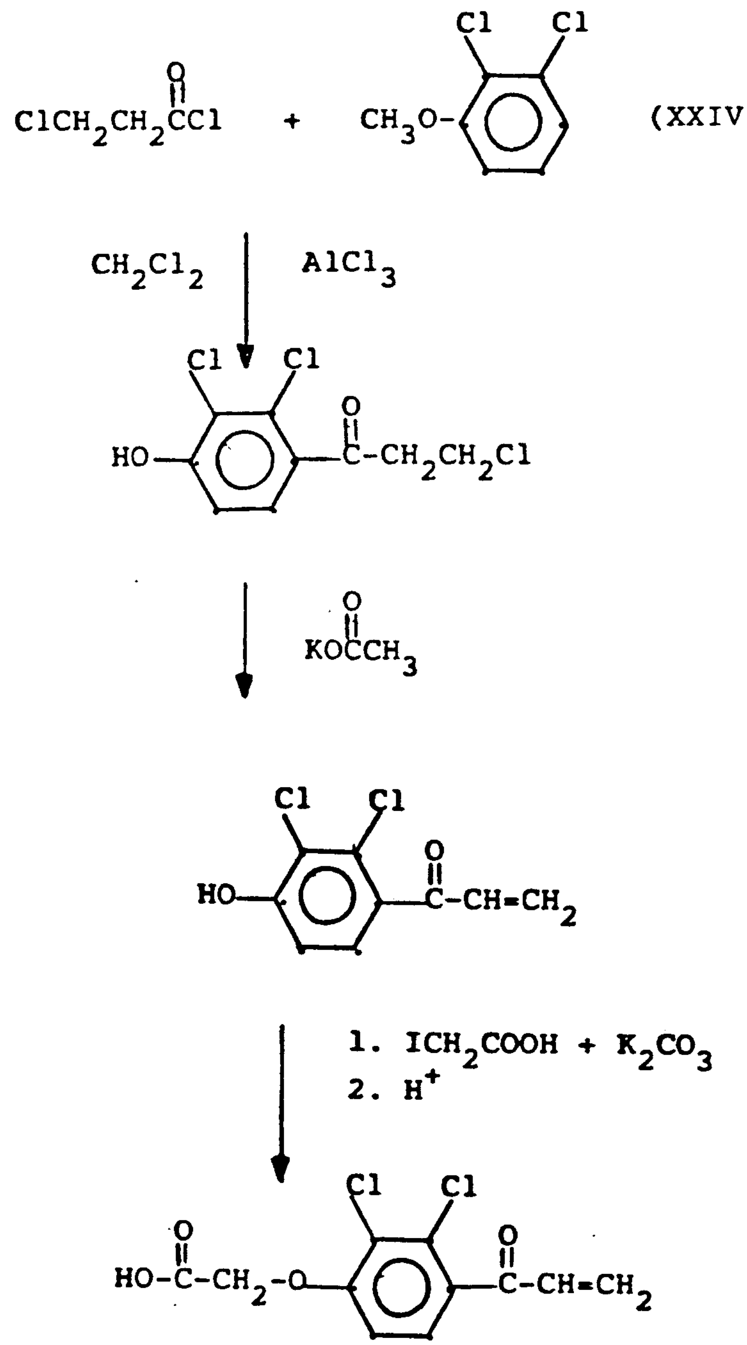

(XXV)
(XXVI)
(XIVd)

A Friedel-Crafts reaction involving 3-chloropropionyl chloride (XXIII) and 2,3-dichloroanisole (XXIV) in the presence of aluminum chloride and an appropriate solvent such as methylene chloride produces the phenol of Formula XXV. By reaction of the compound of formula XXV with potassium acetate in methanol produces the acrylophenone of Formula XXVI. Reaction of the compound of Formula XXVI with iodoacetic acid in acetone in the presence of potassium carbonate at reflux temperature for 15-36 hours produces, upon acidification, the compound of Formula XIVd.

The salts of compounds of Formula IV are also a part of the instant invention. These compounds are prepared by reacting the acids of Formula IV with an appropriate base, for example, alkali metal or alkaline earth bicarbonate, carbonate or alkoxide, an amine, ammonia, an organic quaternary ammonium hydroxide, guanidine and the like.

The reaction is generally conducted in water when alkali metal bicarbonates or carbonates are used, but when alkoxides and the organic bases are used, the reaction may be conducted in an organic solvent, such as ether, ethanol, N,N-dimethylformamide and the like. used, the reaction may be conducted in an organic solvent, such as ether, ethanol, N,N-dimethylformamide and the like.

The preferred salts are the pharmaceutically acceptable salts such as sodium, potassium, ammonium and the like.

The compounds of Structure V wherein $R_3'$ is OH (Structure Va) are prepared as follows:

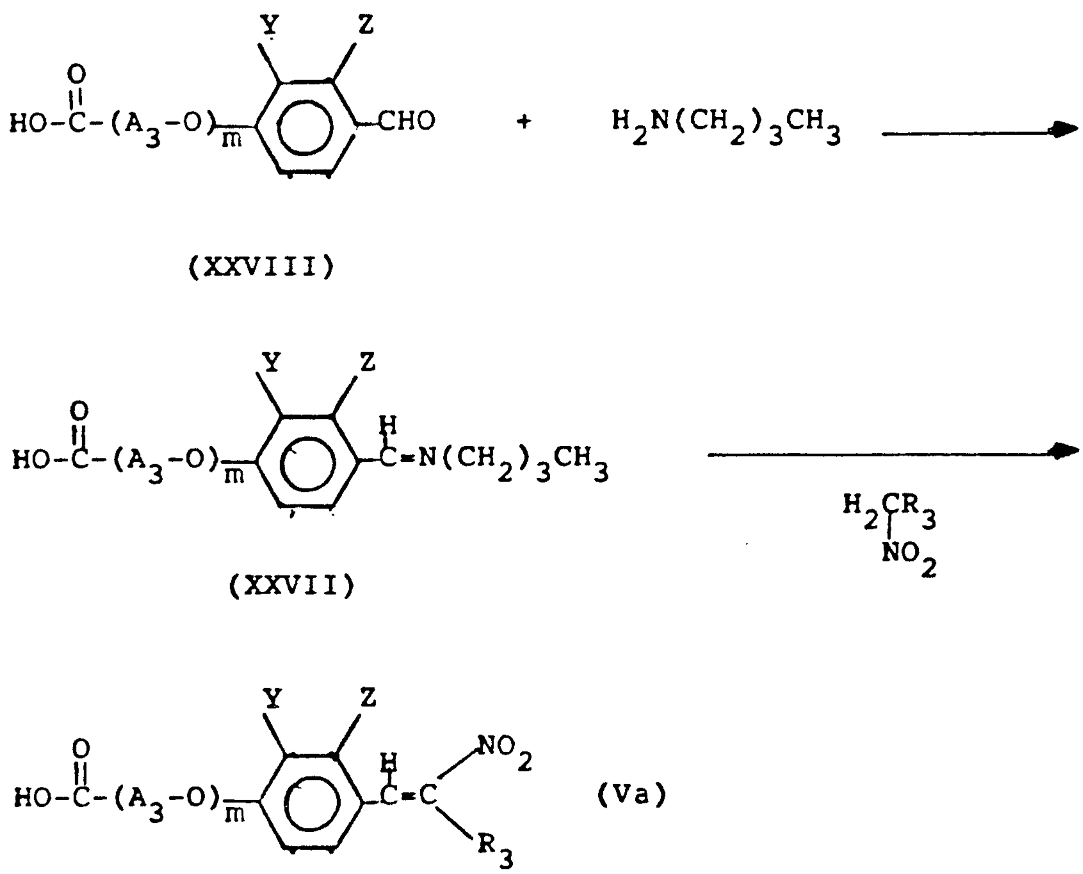

(XXVIII)

(XXVII)

(Va)

A benzaldehyde of Structure XXVIII is heated with butylamine in a solvent such as benzene or toluene until the water that is generated is removed by distillation, thus forming a Schiff base of Structure XXVII. Addition of a nitroalkane of type $R_3CH_2NO_2$ and heating the mixture gave the desired compound of Structure Va.

The intermediate compounds of Formula XXVIIIa (where m = 1) are prepared as follows for compounds where
$A_3$ = $(CH_2)n_3'$ or $-C(CH_2)_3$, where $n_3'$ = 1, 3, 4 or 5.

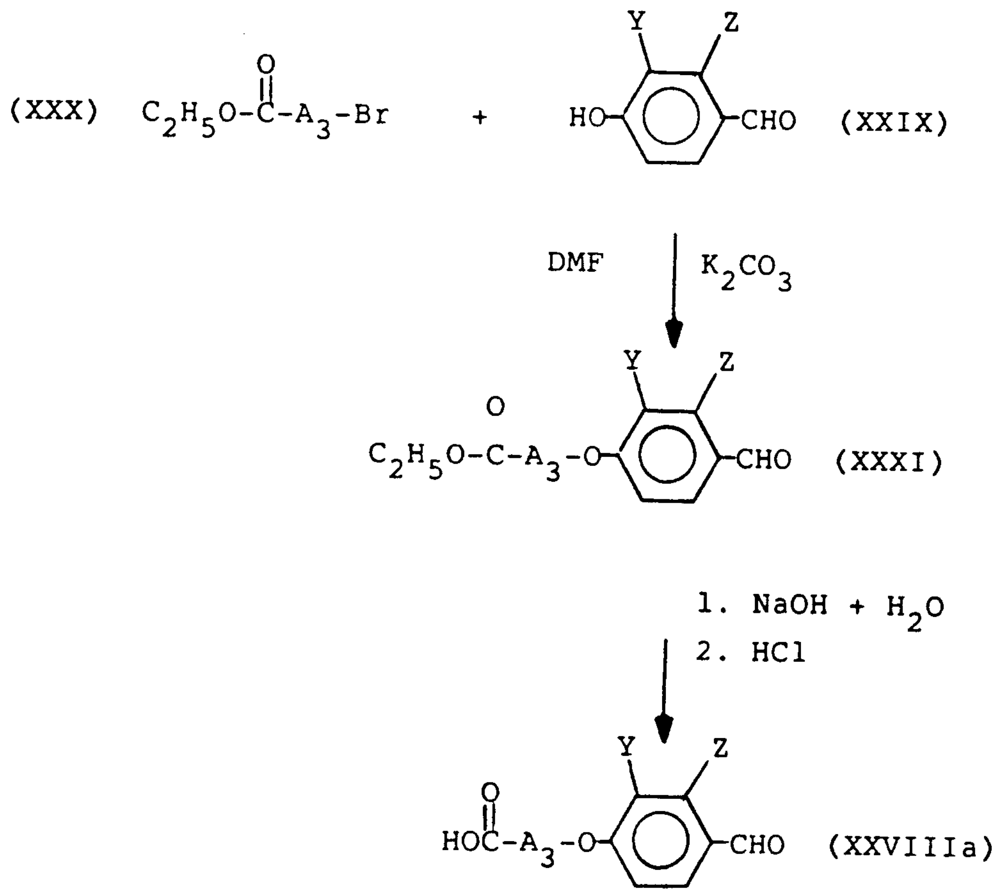


A phenol of Formula XXIX is reacted with a bromo ester of Formula XXX to produce an ester of Formula XXXI. The reaction is conducted in any one of several solvents, such as N,N-dimethylformamide (DMF) or 1-methyl-2-pyrrolidinone in the presence of a base, such as potassium carbonate or sodium carbonate. The reaction temperature is generally of 25 to 100° C, preferably at 55-60° C for a period of one to 48 hours, preferably 10-20 hours.

The esters of Formula XXXI are converted to the corresponding carboxylic acids of Formula XXVIIIa by saponification with aqueous sodium hydroxide followed by acidification. Saponification is conveniently conducted in the presence of the N,N-dimethylformamide solvent used for making the ester. Saponification is generally complete within one to five hours when the reaction temperature is in the range of 75-100° C.

When $A_3$ is $-(CH_2)_2-$ and Y and Z are Cl, the compounds of Formula XXVIIIb are prepared as follows:

(XXXII)

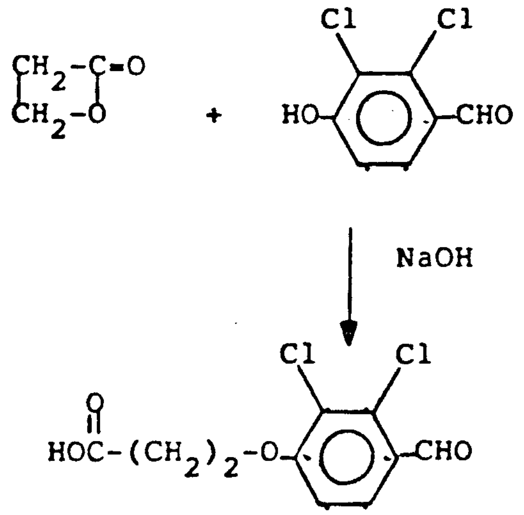


(XXIX)

(XXVIIIb)

Reaction of a phenol of Formula XXIX with β-propiolactone XXXII in water and a base, such as potassium hydroxide or sodium hydroxide gives the product of Formula XXVIIIb. The product is isolated by acidification extracting with ether, extracting the ether layer with aqueous sodium bicarbonate followed by acidification of the aqueous extract with hydrochloric acid.

The compounds of the Structure V wherein $R_3'$ is $HOOC(CH_2)_xCHR_2'$ NH- (Formula Vb) are prepared as follows:

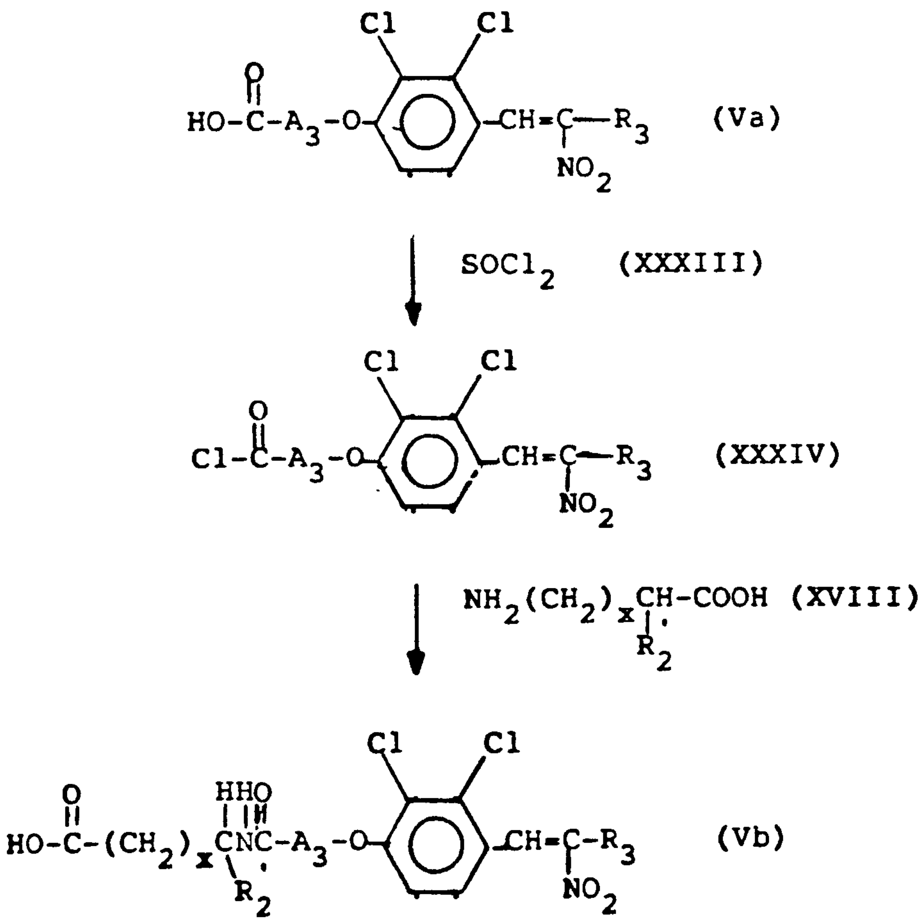

A compound of Formula XXXIV is prepared by treating a compound of Formula Va with thionyl chloride XXXIII or similar reagent in an inert solvent such as benzene, toluene, methylene chloride, carbon tetrachloride and the like. The reaction is conducted at temperatures of 40 ° C to that of the boiling point of the solvent, preferably, if possible, in the range of 60-80 ° C for periods of time of 30 minutes to 6 hours. During this time the hydrogen chloride generated during the reaction is evolved.

The reaction of a compound of Formula XXXIV with two moles of a compound of an amino acid of Formula XVIII gives a compound of Formula Vb.

It should be noted that when $R_2'$ of Formula Vb is other than H, the product of Formula Vb can well be either racemic or the R- or S-enantiomer depending on the structure of Formula XVIII used in the reaction.

The preferred salts are the pharmaceutically acceptable salts such as sodium, potassium, ammonium and the like.

As implied earlier, the compounds of this invention may be administered by a variety of established methods, including intravenously, intramuscularly, subcutaneously, orally and extracorporeally. The precise mode of administration is left to the discretion of the practitioner. The option of extracorporeal administration is unique to the therapeutic approach of this invention. Since the entity to which therapy is administered is the erythrocytes of the patient, blood can be removed from the patient, treated with the drug and returned to the patient after the desired interaction between the drug and the erythrocytes has occurred. Before returning the drug-treated blood, the excess (unreacted drug) may be removed, thus reducing any ancillary effects that this excess might cause.

The compounds of formulae I are utilized by formulating them in a composition such as tablet, capsule or elixir for oral administration. Sterile solutions or suspensions can be used for parenteral administration. About 70 µg to 500 mg of a compound or mixture of compounds of formulae I, or a physiologically acceptable vehicle, carrier, excipient, binder, preservative, stabilizer, flavor, etc. in a unit dosage form as called for by accepted pharmaceutical practice. The amount of active substance in the composition is such that dosage in the range indicated is obtained.

Illustrative of the adjuvants which may be incorporated in tablets, capsules and the like are the following: a binder such as gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose, or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise enhance the pharmaceutical elegance of the preparation. For instance, tablets may be coated with shellac, sugar or the like. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a conventional vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

Since an individual patient may present a wide variation in severity of symptoms and each drug has its unique therapeutic characteristics, it is up to the practitioner to determine the patient's response to treatment and vary the dosages accordingly. A recommended dose range is from 100 µg/kg to 15 mg/kg as a primary dose and then, if necessary a sustaining dose equal to half the primary dose may be administered every 12 to 24 hours.

When the compounds of this invention are employed for extracorporeal treatment of blood, it is generally convenient to use a water soluble salt of the compound which may be made isotonic by addition of sodium chloride. The concentration of the compound which is used for this purpose is generally in the range of 0.1 to 10 mM with a range of 1 to 3 mM being more commonly used.

The following examples are included to illustrate the synthesis of representative compounds of Formula I, the preparation of representative dosage forms and the preparation of sterile solutions for use in the extracorporeal treatment of the blood of patients with sickle cell anemia. It is intended that the specification and examples be considered as exemplary only, with the true scope of the invention being indicated by the claims of the invention.

EXAMPLE 1

Preparation of 3-[2,3-dichloro-4-(2-methylenebutyryl)phenoxy]propionic acid

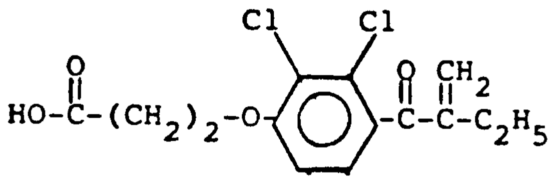

Step A: 2,3-Dichloro-4-(3-butenyloxy)butyrophenone

A mixture of 2,3-dichloro-4-butyrophenone (21 g, 0.090 mole) and potassium carbonate (37 g, 0.27 mole) in N,N-dimethylformamide (100 ml) were stirred and heated to 60 ° C. 4-Bromo-1-butene (26.7 g, 0.2 mole) was added and the mixture stirred and heated at 60 ° -65 ° C for five hours. The reaction mixture was poured into ice water and the mixture extracted with ether. The ether layer was washed with water and then with brine and finally dried over $MgSO_4$. The ether was removed by evaporation at reduced pressure to give 25 g of 2,3-dichloro-4-(3-butenyloxy)-butyrophenone as confirmed by elemental analysis.

Step B: 3-(2,3-Dichloro-4-butyrylphenoxy)propionic acid

2,3-Dichloro-4-(3-butenyloxy)butyrophenone (31.5 g, 0.11 mole) and benzyltriethylammonium chloride (2.3 g) in methylene chloride (350 ml) was added dropwise to a solution of potassium permanganate (45.8 g) in water (900 ml) at 5 ° C. The mixture was then stirred at 20 ° C for 1.5 hours after which tests showed that the oxidation was complete. Sodium bisulfite and dilute hydrochloric acid was added until the color due to $KMnO_4$ disappeared. The mixture was filtered to remove a small amount of solid and the methylene chloride layer from the filtrate was separated. This layer was washed with water and then extracted with a dilute sodium hydroxide solution. The aqueous layer was poured into ice water containing dilute hydrochloric acid. The solid that separated was removed by filtration, washed with water, dried and recrystallized to give 9 g of 3-(2,3-dichloro-4-butyrylphenoxy)propionic acid, m.p. 138-139 ° .

| Anal. Calc'd for $C_{13}H_{14}Cl_2O_4$: | | |
|---|---|---|
| | C, 51.17; | H, 4.62. |
| Found: | C, 51.11; | H, 4.74. |

Step C: 3-[2,3-Dichloro-4-(2-methylenebutyryl)phenoxy]propionic acid

3-(2,3-Dichloro-4-butyrylphenoxy)propionic acid (6.65 g, 0.022 mole), dimethylamine hydrochloride (12 g, 0.15 mole), paraformaldehyde (2.3 g, 0.077 equiv.) and acetic acid (1 ml) were mixed and stirred while heating on a steam bath for 2.5 hours. N,N-dimethylformamide (25 ml) was added to the mixture and stirring and heating continued for another 1.5 hours. The mixture was poured into ice water (600 ml) and the precipitate that formed removed by filtration, washed with water and dried. After recrystallization from butyl chloride, 58 g 3-[2,3-dichloro-4-(2-methylenebutyryl)phenoxy]propionic acid remained.

| Anal. Calc'd for $C_{14}H_{14}Cl_2O_4$: | | |
|---|---|---|
| | C, 53.01; | H, 4.45. |
| Found: | C, 52.88; | H, 4.50. |

EXAMPLE 2

Preparation of 4-[2,3-dichloro-4-(2-methylenebutyryl)phenoxy]butyric acid

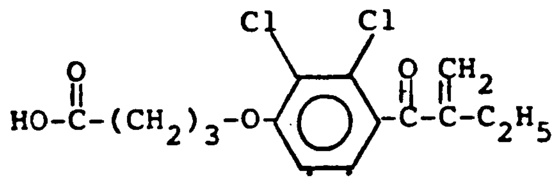

Step A: 4-(2,3-Dichloro-4-butyrylphenoxy)butyric acid

A mixture of 2,3-dichloro-4-butyrylphenol (69.9 g, 0.30 mole), potassium carbonate (75 g, 0.54 mole) and ethyl 4-bromobutyrate (87.8 g, 0.45 mole) in N,N-dimethylformamide (900 ml of sieve-dried material) were stirred and heated at 55-60 ° C for 16.5 hours. The mixture was then treated with 10 normal sodium hydroxide solution (120 ml) and water (600 ml) and stirring and heating at 100 ° C maintained for 2 hours. The solution was cooled and poured into ice water (4 liters) containing concentrated hydrochloric acid (250 ml). The solid that separated was removed by filtration, washed with water and dried. The product (88 g) was recrystallized from butyl chloride (500 ml) to give 78.6 g of 4-(2,3-dichloro-4-butyrylphenoxy)butyric acid, m.p. 111-113 ° C.

| Anal. Calc'd for $C_{14}H_{16}Cl_2O_4$: | | | |
|---|---|---|---|
| | C, 52.68; | H, 5.05; | Cl, 22.21. |
| Found: | C, 52.62; | H, 5.09; | Cl, 22.17. |

Step B: 4-[2,3-Dichloro-4-(2-methylenebutyryl)phenoxy]butyric acid

4-(2,3-Dichloro-4-butyrylphenoxy)butyric acid (71.8 g, 0.225 mole), paraformaldehyde (16.5 g, 0.549 equivalent), dimethylamine hydrochloride (82.0 g, 1.0 mole) and acetic acid (5.5 ml) were united and stirred on a steam bath for 7 hours. N,N-dimethylformamide (172 ml) was added and stirring and heating continued for another hour. The solution was then poured into cold l normal hydrochloric acid (1.5 liters) with stirring. The product that separated slowly solidified. This material was pulverized, filtered, washed with water and dried to give 70.8 g of product. This material was recrystallized from butyl chloride to give 4-[2,3-dichloro-4-(2-methylenebutyryl)phenoxy]butyric acid melting at 87-89 ° C.

| Anal. Calc'd for $C_{15}H_{16}Cl_2O_4$: | | | |
|---|---|---|---|
| | C, 54.40; | H, 4.87; | Cl, 21.41. |
| Found: | C, 54.03; | H, 4.98; | Cl, 21.60. |

EXAMPLE 3

Preparation of 4-[2,3-dichloro-4-(2-methylenevaleryl)phenoxy]butyric acid

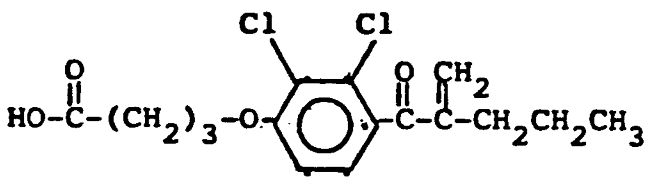

Step A: 4-(2,3-Dichloro-4-valerylphenoxy)butyric acid

By conducting the reaction as described in Example 2, Step A, except that an equimolar amount of 2,3-dichloro-4-valerylphenol was substituted for the 2,3-dichloro-4-butyrylphenol, there was obtained 4-(2,3-dichloro-4-valerylphenoxy)butyric acid, m.p. 92-94° C.

| Anal. Calc'd. for $C_{15}H_{18}Cl_2O_4$: | | |
|---|---|---|
| | C, 54.07; | H, 5.45. |
| Found: | C, 54.63; | H, 5.63. |

Step B: 4-[2,3-Dichloro-4-(2-methylenevaleryl)phenoxy]butyric acid

By conducting the reaction as described in Example 2, Step B, except that an equimolar amount of 4-(2,3-dichloro-4-valerylphenoxy)butyric acid was substituted for the 4-(2,3-dichloro-4-butyrylphenoxy)butyric acid, there was obtained 4-[2,3-dichloro-4-(2-methylenevaleryl)phenoxy]butyric acid, m.p. 85-87° C.

| Anal. Calc'd for $C_{16}H_{18}Cl_2O_4$: | | |
|---|---|---|
| | C, 55.67; | H, 5.26. |
| Found: | C, 56.02; | H, 5.34. |

EXAMPLE 4

Preparation of 5-[2,3-dichloro-4-(2-methylenebutyryl)phenoxy]valeric acid

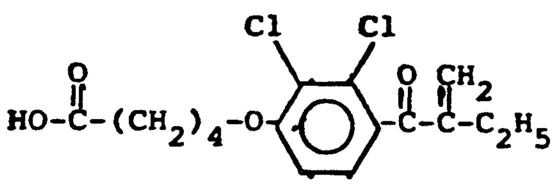

Step A: 5-(2,3-Dichloro-4-butyrylphenoxy)valeric acid

A mixture of 2,3-dichloro-4-butyrylphenol (7 g, 0.03 mole), potassium carbonate (7.5 g, 0.054 mole) and ethyl 5-bromovalerate (9.5 g, 0.045 mole) in N,N-dimethylformamide (60 ml) were stirred and heated at 60° C for 3 hours. The mixture was treated with 10 normal sodium hydroxide (12 ml) and water (50 ml), then stirred and heated on a steam bath for 2.5 hours. The solution was poured into ice water containing

hydrochloric acid. The solid that separated was removed by filtration, washed with water and dried. The product (9.8 g) was recrystallized from butyl chloride to give 5-(2,3-dichloro-4-butyrylphenoxy)valeric acid, m.p. 102-103 ° C.

| Anal. Calc'd for $C_{15}H_{18}Cl_2O_4$: | | |
|---|---|---|
| | C, 54.07; | H, 5.45. |
| Found: | C, 54.09; | H, 5.38. |

Step B: 5-[2,3-Dichloro-4-(2-methylenebutyryl)phenoxy]valeric acid

5-(2,3-Dichloro-4-butyrylphenoxy)valeric acid (9.8 g, 0.029 mole), dimethylamine hydrochloride (15 g, 0.184 mole), paraformaldehyde (2.8 g, 0.09 equivalent) and acetic acid (1 ml) were united and stirred and heated on a steam bath for 3 hours. N,N-dimethylformamide (30 ml) was added and stirring and heating continued for another two hours. The reaction mixture was then poured into ice water and the solid that separated was removed by filtration, washed with water and dried. After recrystallization from butyl chloride the 5-[2,3-dichloro-(2-methylenebutyryl)phenoxy]valeric acid (5.4 g) melted at 107-109 ° C.

| Anal. Calc'd for $C_{16}H_{18}Cl_2O_4$: | | |
|---|---|---|
| | C, 55.66; | H, 5.26. |
| Found: | C, 55.62; | H, 5.31. |

EXAMPLE 5

Preparation of 6-[2,3-dichloro-4-(2-methylenebutyryl)phenoxy]hexanoic acid

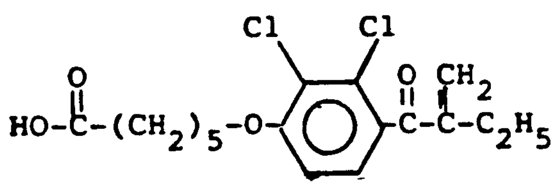

Step A: 6-(2,3-Dichloro-4-butyrylphenoxy)hexanoic acid

A mixture of 2,3-dichloro-4-butyrylphenol (7 g, 0.03 mole), potassium carbonate (7.5 g, 0.054 mole) and ethyl 6-bromohexanoate (10.25 g, 0.045 mole) in N,N-dimethylformamide (60 ml) was stirred and heated at 65 ° C for 2.5 hours. The mixture was treated with 10 normal sodium hydroxide (12 ml) and water (50 ml), then stirred and heated on a steam bath for 2.5 hours. The reaction mixture was poured into ice water containing hydrochloric acid. The solid that separated was removed by filtration, washed with water and dried to give 10 g of product. Recrystallization from butyl chloride gave 6-(2,3-dichloro-4-butyrylphenoxy)-hexanoic acid, m.p. 94-95 ° C.

| Anal. Calc'd for $C_{16}H_{20}Cl_2O_4$: | | |
|---|---|---|
| | C, 55.34; | H, 5.81. |
| Found: | C, 55.39; | H, 5.84. |

Step B: 6-[2,3-Dichloro-4-(2-methylenebutyryl)phenoxy)hexanoic acid

A mixture of 6-(2,3-dichloro-4-butyrylphenoxy)hexanoic acid (9.5 g, 0.026 mole), dimethylamine hydrochloride (15 g, 0.184 mole), paraformaldehyde (2.8 g, 0.09 equiv.) and acetic acid (1 ml) were united and stirred and heated on a steam bath for two hours. The reaction mixture was treated with N,N-dimethylformamide (30 ml) and stirring and heating continued for another hour. The reaction mixture was poured into ice water and the solid that separated was removed by filtration, washed with water and dried. The yield was 9.2 g after recrystallization from butyl chloride the 6-[2,3-dichloro-4-(2-methylenebutyryl)phenoxy]-hexanoic acid melted at 106-107 ° C.

| Anal. Calc'd for $C_{17}H_{20}Cl_2O_4$: | | |
|---|---|---|
| | C, 56.84; | H, 5.61. |
| Found: | C, 57.20; | H, 5.75. |

EXAMPLE 6

Preparation of 1-[2,3-dichloro-4-(2-methylenebutyryl)phenoxy]cyclobutane-1-carboxylic acid

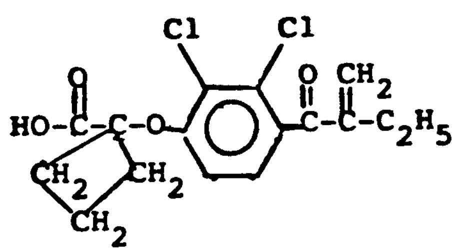

Step A: 1-(2,3-Dichloro-4-butyrylphenoxy)cyclobutane-1-carboxylic acid

A mixture of 2,3-dichloro-4-butyrylphenol (7 g, 0.03 mole), ethyl α-bromocyclobutanecarboxylate (10.23 g, 0.049 mole) and potassium carbonate (7.5 g, 0.054 mole) in N,N-dimethylformamide (60 ml) was stirred and heated at 65 ° C for 18 hours. The reaction mixture was poured into ice water and the resulting mixture was extracted with ether. The ether extract was washed with a 1% sodium hydroxide solution, then with water and finally dried over $MgSO_4$. The ether was evaporated in vacuo and the residue treated with acetic acid (60 ml) and 5% hydrochloric acid (15 ml). The mixture was stirred and refluxed overnight. The solvents were evaporated in vacuo. The residue was chromatographed over a column containing silica gel (140 g) using methylene chloride/tetrahydrofuran/acetic acid 50/1/1 as the eluent. The pertinent fractions were united, treated with toluene and evaporated in vacuo to obtain 1-(2,3-dichloro-4-butyrylphenoxy)cyclobutane-1-carboxylic acid as a liquid whose identity was confirmed by NMR and purity by thin layer chromatography.

Step B: 1-[2,3-Dichloro-4-(2-methylenebutyryl)phenoxy]cyclobutane-1-carboxylic acid

A mixture of 1-(2,3-dichloro-4-butyrylphenoxy)cyclobutane-1-carboxylic acid (4 g, 0.012 mole), dimethylamine hydrochloride (5.5 g, 0.067 mole), paraformaldehyde (1.2 g, 0.04 equiv.) and acetic acid (0.3 ml) were stirred and heated on a steam bath for 2.25 hours. N,N-dimethylformamide (30 ml) was added and stirring and heating was continued for another 2 hours. The mixture was poured into ice water and extracted with ether. The ether layer was washed with water and then with brine and finally dried over $MgSO_4$. The solution was treated with p-xylene and the solvents were removed in vacuo. The resulting residue was recrystallized from hexane to give 1-[2,3-dichloro-4-(2-methylenebutyryl)phenoxy]cyclobutane-1-carboxylic acid, m.p. 120 ° C.

| Anal. Calc'd for $C_{16}H_{16}Cl_2O_4$: | | |
|---|---|---|
| | C, 55.99; | H, 4.70. |
| Found: | C, 56.09; | H, 4.80. |

EXAMPLE 7

Preparation of 1-[2,3-dichloro-4-(2-methylenevaleryl)phenoxy]cyclobutane-1-carboxylic acid

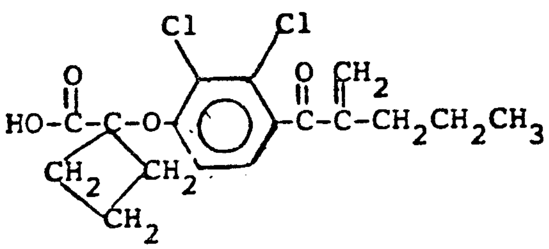

Step A: 1-(2,3-Dichloro-4-valerylphenoxy)cyclobutane1-carboxylic acid

By conducting the reaction as described in Example 6, Step A, except that an equimolar amount of 2,3-dichloro-4-valerylphenol was used in place of the 2,3-dichloro-4-butyrylphenol, there was obtained 1-(2,3-dichloro-4-valerylphenoxy)cyclobutane-1-carboxylic acid.

Step B: 1-[2,3-Dichloro-4-(2-methylenevaleryl)phenoxy]cyclobutane-1-carboxylic acid

By conducting the reaction as described in Example 6, Step B, except that an equimolar amount of 1-(2,3-dichloro-4-valerylphenoxy)cyclobutane-1-carboxylic acid was used in place of the 1-(2,3-dichloro-4-butyrylphenoxy)cyclobutane-1-carboxylic acid, there is obtained 1-[2,3-dichloro-4-(2-methylenevaleryl)-phenoxy]cyclobutane-1-carboxylic acid, m.p. 105-106 ° C.

| Anal. Calcd. for $C_{17}H_{18}Cl_2O_4$: | | |
|---|---|---|
| | C, 57.16; | H, 5.05. |
| Found: | C, 57.46; | H, 5.15. |

EXAMPLE 8

Preparation of 2-[2,3-dichloro-4-(2-methylenebutyryl)phenoxy]-2-methylpropionic acid

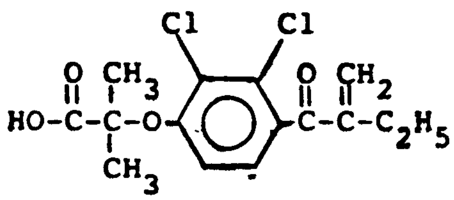

Step A: 2-(2,3-Dichloro-4-butyrylphenoxy)-2-methylpropionic acid

A mixture of 2,3-dichloro-4-butyrylphenol (15 g, 0.064 mole), acetone (140 ml) and sodium hydroxide (14.5 g, 0.36 mole) was stirred and heated to reflux. Chloroform (12 g, 0.1 mole) was added to the mixture dropwise over a period of 15 minutes. Refluxing was continued for 3 hours and the acetone was evaporated in vacuo. The residue was treated with ice water containing hydrochloric acid. The product was extracted with ether. The ether extract was washed with water, then with brine and finally dried over $MgSO_4$. The ether was evaporated in vacuo and the residue chromatographed on a column containing silica gel (320 g) and eluted with methylene chloride/tetrahydrofuran/acetic acid 50/1/1. The appropriate fractions were combined and evaporated in vacuo to give 10 g of 2-(2,3-dichloro-4-butyrylphenoxy)-2-methylpropionic acid which was identified by NMR and the purity confirmed by thin layer chromatography.

Step B: 2-[2,3-Dichloro-4-(2-methylenebutyryl)phenoxy)-2-methylpropionic acid

A mixture of 2-(2,3-dichloro-4-butyrylphenoxy)-2-methylpropionic acid (10 g, 0.031 mole), dimethylamine hydrochloride (15 g, 0.18 mole), paraformaldehyde (3 g, 0.10 equiv.) and acetic acid (0.6 ml) was stirred and heated on a steam bath for two hours. N,N-dimethylformamide (30 ml) was added and stirring and heating was continued for another two hours. The mixture was poured into ice water which was then extracted with ether. The ether extract was washed with water, then brine and finally dried over $MgSO_4$. The ether was evaporated in vacuo and the residue recrystallized from cyclohexane to give 6.3 g 2-[2,3-dichloro-4-(2-methylenebutyryl)phenoxy]-2-methylpropionic acid, m.p. 88-89 °C.

| Anal. Calc'd for $C_{15}H_{16}Cl_2O_4$: | | |
|---|---|---|
| | C, 54.40; | H, 4.87. |
| Found: | C, 54.71; | H, 5.05. |

EXAMPLE 9

N-{[2,3-Dichloro-4-(2-methylenebutyryl)phenoxy]acetyl}glycine

[2,3-Dichloro-4-(2-methylenebutyryl)phenoxy)acetic acid (18.19 g, 0.06 mole) was dissolved in dry benzene (45 ml) and thionyl chloride (19.04 g, 0.12 mole) and the mixture stirred and refluxed for 75 minutes. The volatile materials were removed by evaporation at reduced pressure and the residue, which consisted of [2,3-dichloro-4-(2-methylenebutyryl)phenoxy]acetyl chloride, was dissolved in dry tetrahydrofuran (50 ml).

To a stirring suspension of finely ground glycine (6.01 g, 0.08 mole) in tetrahydrofuran (50 ml) was added, dropwise, the solution of [2,3-dichloro-4-(2-methylenebutyryl)phenoxy]acetyl chloride over a period of 15 minutes. The mixture was stirred at ambient temperature for 2.25 hours and then at reflux for one hour. The mixture was cooled, filtered and washed with tetrahydrofuran. The solid was suspended in water (60 ml), filtered, washed with water and dried to give 7.15 g of N-{[2,3-dichloro-4-(2-methylenebutyryl)-phenoxy]acetyl}glycine. Upon recrystallization from isopropyl alcohol, the product melted at 201.5-202.5 °C.

| Anal. Calc'd for $C_{15}H_{15}Cl_2NO_5$: | | | |
|---|---|---|---|
| | C, 50.02; | H, 4.20; | N, 3.89. |
| Found: | C, 49.73; | H, 4.45; | N, 3.81. |

EXAMPLE 10

N-{[2,3-Dichloro-4-(2-methylenebutyryl)phenoxy]acetyl}-S(+)-alanine

By carrying out the reaction essentially as described in Example 9, except that the glycine was replaced by an equimolar quantity of L-alanine, there was obtained N-{[2,3-dichloro-4-(2-methylenebutyryl)-phenoxy]acetyl}-S(+)-alanine, 16.5 g, m.p. 202.5-203.5 °C.

| Anal. Calc'd for $C_{16}H_{17}Cl_2NO_5$: | | | |
|---|---|---|---|
| | C, 51.35; | H, 4.58; | N, 3.74. |
| Found: | C, 51.27; | H, 4.55; | N, 3.71. |

EXAMPLE 11

N-{[2,3-Dichloro-4-(2-methylenebutyryl)phenoxy]acetyl}-S(+)-leucine

By carrying out the reaction essentially as described in Example 9, except that the glycine is replaced by an equimolar quantity of S(+)leucine, there is obtained N-{[2,3-dichloro-4-(2-methylenebutyryl)phenoxy]-acetyl}-S(+)-leucine.

EXAMPLE 12

N-{[2,3-Dichloro-4-(2-methylenebutyryl)phenoxy]acetyl}-S(+)-serine

By carrying out the reaction essentially as described in Example 9, except that the glycine is replaced by an equimolar quantity of L-serine, there is obtained N-{[2,3-dichloro-4-(2-methylenebutyryl)phenoxy]-acetyl}-S(+)-serine.

EXAMPLE 13

N-{[2,3-Dichloro-4-(2-methylenebutyryl)phenoxy]acetyl}-S(-)-phenylalanine

By carrying out the reaction essentially as described in Example 9, except that the glycine is replaced by an equimolar quantity of L-phenylalanine, there is obtained N-{([2,3-dichloro-4-(2-methylenebutyryl)-phenoxy]acetyl))-S(-)-phenylalanine.

EXAMPLE 14

N-{4-[2,3-Dichloro-4-(2-methylebutyryl)phenoxy]butyryl}glycine

By carrying out the reaction essentially as described in Example 9, except that the [2,3-dichloro4-(2-methylenebutyryl)phenoxy]acetic acid is replaced by an equimolar quantity of 4-[2,3-dichloro-4-(2-methylenebutyryl)phenoxy]butyric acid, there )s obtained N-{4-[2,3-dichloro-4-(2-methylenebutyryl)-phenoxy]butyryl}glycine.

EXAMPLE 15

N-{1-[2,3-Dichloro-4-(2-methylenebutyryl)phenoxy]cyclobutane-1-carbonyl}glycine

By carrying out the reaction essentially as described in Example 9, except that the [2,3-dichloro-4-(2-methylenebutyryl)phenoxy]acetic acid is replaced by an equimolar quantity of 1-[2,3-dichloro-(2-methylenebutyryl]cyclobutane-1-carboxylic acid, there is obtained N-{1-[2,3-dichloro-(2-methylenebutyryl)-phenoxy]cyclobutane-1-carbonyl}glycine.

EXAMPLE 16

N-{3-[2,3-Dichloro-4-(2-methylenebutyryl)phenoxy]propionyl}glycine

By carrying out the reaction essentially as described in Example 9, except that the [2,3-dichloro-4-(2-methylenebutyryl)phenoxy]acetic acid is replaced by an equimolar quantity of 3-[2,3-dichloro-(2-methylenebutyryl)phenoxy]propionic acid, there is obtained N- 3-[2,3-dichloro-4-(2-methylenebutyryl)-phenoxy]propionyl glycine.

EXAMPLE 17

N-[(2,3-Dichloro-4-acryloylphenoxy)acetyl]glycine

Step A: 2,3-Dichloro-4-(3-chloropropionyl)phenol

A stirred mixture of 2,3-dichloroanisole (71 g, 0.6 mole) and 3-chloropropionyl chloride (76.5 g, 0.6 mole) in dry methylene chloride (300 ml) was cooled to 5°C and aluminum chloride (80 g, 0.6 mole) added portionwise over a period of 30 minutes. After stirring an additional 2 hours, the methylene chloride was removed by distillation. More methylene chloride (150 ml) was added and removed by distillation.

The residue was treated with more methylene chloride (200 ml) and aluminum chloride (80 g, 0.6 mole) and the mixture stirred and refluxed for 2.5 hours. The reaction mixture was cooled and poured into a mixture of ice and concentrated hydrochloric acid (70 ml). The mixture was extracted with ether and the organic layer washed with water, dried over $MgSO_4$, filtered and the solvents removed by evaporation in vacuo. The residue was treated with benzene to give 28 g of a solid which was removed by filtration. Treatment of the filtrate with cyclohexane gave another 6.5 g of product. The combined products were recrystallized from benzene to give 2,3-dichloro-4-(3-chloropropionyl)phenol, m.p. 109-111°C.

| Anal. Calc'd for $C_9H_7Cl_3O_2$: | | |
|---|---|---|
| | C, 42.63; | H, 2.78. |
| Found: | C, 42.99; | H, 2.77. |

Step B: 2,3-Dichloro-4-acryloylphenol

A solution of 2,3-dichloro-4-(3-chloropropionyl)phenol (8.4 g, 0.033 mole) in methanol (30 ml) and potassium acetate (3.5 g, 0.033 mole) in methanol (30 ml) were united and refluxed for 20 minutes. The mixture was treated with chloroform (200 ml), washed with water, dried over $MgSO_4$, filtered and the solvent removed from the filtrate by evaporation in vacuo. The residue was recrystallized from carbon tetrachloride to give 6 g of 2,3-dichloro-4-acryloylphenol, m.p. 135-137°C.

| Anal. Calc'd for $C_9H_6Cl_2O_2$: | | | |
|---|---|---|---|
| | C, 49.80; | H, 2.79; | Cl, 32.67. |
| Found: | C, 49.43; | H, 2.94; | Cl, 32.83. |

Step C: (2,3-Dichloro-4-acryloylphenoxy)acetic acid

2,3-Dichloro-4-acryloylphenol (4.85 g, 0.0224 mole), iodoacetic acid (4.5 g, 0.24 mole), potassium carbonate (3.33 g, 0.024 mole) and acetone (200 ml) were united, stirred and refluxed for 20 hours. The reaction mixture was cooled and the solid removed by filtration, dissolved in water (100 ml) and acidified to a pH of 1 with concentrated hydrochloric acid. The material that separated was extracted with chloroform, the organic layer removed, washed with water, dried over $MgSO_4$, filtered and the solvent removed by evaporation in vacuo. The residue was recrystallized from carbon tetrachloride to give 2.2 g of (2,3-dichloro-4-acryloylphenoxy)acetic acid, m.p. 111-113 °C.

| Anal. Calc'd for $C_{11}H_8Cl_2O_4$: | | | |
|---|---|---|---|
| | C, 48.03; | H, 2.93; | Cl, 25.78. |
| Found: | C, 47.96; | H, 3.00; | Cl, 25.82. |

Step D: N-[(2,3-Dichloro-4-acryloylphenoxy)acetyl]glycine

By carrying out the reaction essentially as described in Example 9, except that the [2,3-dichloro-4-(2-methylenebutyryl)phenoxy]acetic acid is replaced by an equimolar quantity of (2,3-dichloro-4-acryloylphenoxy)acetic acid, there is obtained N-[(2,3-dichloro-4-acryloylphenoxy)acetyl]glycine.

EXAMPLE 18

N-{1-[2,3-Dichloro-4-(2-methylenevaleryl)phenoxy]cyclobutane-1-carbonyl}glycine

By carrying out the reaction essentially as described in Example 9, except that the [2,3-dichloro-4-(2-methylenebutyryl)phenoxy]acetic acid is replaced by an equimolar quantity of 1-[2,3-dichloro-4-(2-methylene-valeryl)phenoxy]cyclobutane-1-carboxylic acid, there is obtained N-{1-[2,3-dichloro-4-(2-methylene- valeryl)phenoxy]cyclobutane-1-carbonyl}glycine.

EXAMPLE 19

N-{1-[2,3-Dichloro-4-(2-methylenebutyryl)phenoxy]cyclobutane-1-carbonyl}-S(+)-alanine

By carrying out the reaction essentially,as described in Example 9, except that the [2,3-dichloro-4-(2-methylenebutyryl)phenoxy]acetic acid is replaced by an equimolar quantity of 1-[2,3-dichloro-4-(2-methylenebutyryl)phenoxy]cyclobutane-1-carboxylic acid and the glycine is replaced by an equimolar quantity of L-alanine, there is obtained N-{1-[2,3-dichloro-4-(2-methylenebutyryl)phenoxy]cyclobutane-1-carbonyl}-S(+)-alanine.

EXAMPLE 20

N-{1-[2,3-Dichloro-4-(2-methylenebutyryl)phenoxy]cyclobutane-1-carbonyl}-3-aminopropionic acid

By carrying out the reaction essentially as described in Example 9, except that the [2,3-dichloro-4-(2-methylenebutyryl)phenoxy)acetic acid is replaced by an equimolar quantity of 1-[2,3-dichloro-4-(2-methylenebutyryl)phenoxy]cyclobutane-1-carboxylic acid and the glycine is replaced by an equimolar quantity of $\beta$-alanine, there is obtained N-{1-[2,3-dichloro-4-(2-methylenebutyryl)phenoxy]cyclobutane-1-carbonyl}-3-aminopropionic acid.

EXAMPLE 21

(E)-4-[2,3-dichloro-4-(2-nitro-1-butenyl)phenoxy]butyric acid

Step A: 4-(2,3-dichloro-4-formylphenoxy)butyric acid

A mixture of 2,3-dichloro-4-formylphenol (5.73 g., 0.03 mole), ethyl 4-bromobutyrate (11.7 g., 0.06 mole) and potassium carbonate (8.28 g., 0.06 mole) in dimethylformamide (25 ml.) is heated at 50-60° C for 1 1/2 hours. Water (50 ml.) is added and the mixture is extracted with ether. The ether extract is dried and evaporated. To the residue is added a solution composed of 48 ml. of 40% sodium bisulfite and 12 ml. of alcohol. The precipitated bisulfite addition compound then is washed with alcohol and ether. The solid is suspended in water heated in a steam bath. An oily layer of the free aldehyde is formed. The hot water then is decanted and the oily layer is extracted with ether and the ether evaporated. The residue is heated to 80° C in a mixture of potassium hydroxide (2.8 g.), methanol (30 ml.) and water (5 ml.), the resulting solution is evaporated to dryness and the residue dissolved in water. Upon acidification a solid separates. The solid is crystallized from benzene to obtain 2.9 g. of 4-(2,3-dichloro-4-formylphenoxy)butyric acid, M.P. 147-148° C.

Analysis for $C_{11}H_{11}Cl_2O_4$:

Calculated, C, 47.67; H, 3.64; Cl, 25.59. Found: C, 47.70; H, 3.60; Cl, 25.65.

Step B: 4-[2,3-dichloro-4-(2-nitro-1-butenyl)phenoxy]butyric acid

The 4-(2,3-dichloro-4-formylphenoxy)butyric acid (2.7 g., 0.01 mole) and n-butylamine (2.2 g., 0.03 mole), in benzene, are refluxed for three hours or until no more water is evolved. The benzene is evaporated and then acetic acid (12 ml.) and 1-nitropropane (3.6 g., 0.04 mole) are added. The mixture is heated to boiling and then poured onto ice. The yellow solid that separates is crystallized from ethanol to obtain 1.3 g. of 4-[2,3-dichloro-4-(2-nitro-1-butenyl)phenoxy]butyric acid , M.P. 155-156.5° C.

Analysis for $C_{14}H_{15}Cl_2NO_5$:

Calculated, C, 48.29; H, 4.50; N, 4.02. Found: C, 48.51; H, 4.42; N, 4.30.

EXAMPLE 22

(E)-3-[2,3-dichloro-4-(2-nitro-1-propenyl)phenoxy]propionic acid

Step A: 3-(2,3-dichloro-4-formylphenoxy)propionic acid

2,3-dichloro-4-hydroxybenzaldehyde (38.2 g., 0.2 mole) is dissolved in a 10% sodium hydroxide solution (200 ml.). The solution is heated to boiling and $\beta$-propiolactone (144 g., 2.0 moles) is added dropwise at such a rate as to keep the solution boiling. During the addition 10% sodium hydroxide solution is added in portions to maintain an alkaline mixture. Then the solution is cooled and acidified. The precipitated material is dissolved in ether and the product is extracted into a 5% sodium bicarbonate solution. Acidification of the aqueous solution precipitates 3-(2,3-dichloro-4-formylphenoxy)propionic acid, which is purified by recrystallization from ethyl acetate.

Step B: (E)-3-[2,3-dichloro-4-(2-nitro-1-propenyl)phenoxy]propionic acid.

By following substantially the procedure described in Example 21, Step B, except that the 4-(2,3-dichloro-4-formylphenoxy)butyric acid used therein is replaced by an equimolar quantity of 3-(2,3-dichloro-4-formylphenoxy)propionic acid, and there is obtained (E)-3-[2,3-dichloro-4-(2-nitro-1-propenyl)phenoxy]-propionic acid.

EXAMPLE 23

(E)-4-[2,3-dichloro-4-(2-nitro-1-pentenyl)phenoxy]butyric acid

By following substantially the procedure described in Example 21, Step B, except that the 1-nitropropane used therein is replaced by an equimolar quantity of 1-nitrobutane, and there is obtained 4-[2,3-dichloro-4-(2-nitrol-1-pentenyl)phenoxy]butyric acid.

EXAMPLE 24

(E)-5-[2,3-dichloro-4-(2-nitro-1-butenyl)phenoxy]pentanoic acid

Step A: 5-(2,3-dichloro-4-formylphenoxy)pentanoic acid

By following substantially the procedure described in Example 21, Step A, except that the ethyl 4-bromobutyrate used therein is replaced by an equimolar quantity of ethyl 5-bromovalerate, and there is obtained 5-(2,3-dichloro-4-formylphenoxy) pentanoic acid.

Step B: (E)-5-[2,3-dichloro-4-(2-nitro-1-butenyl)phenoxy]pentanoic acid.

By following substantially the procedure described in Example 21, Step B, except that the 4-(2,3-dichloro-4-formylphenoxy)butyric acid, used therein is replaced by an equimolar quantity of 5-(2,3-dichloro-4-formylphenoxy)pentanoic acid, and there is obtained (E)-5-[2,3-dichloro-4-(2-nitro-1-butenyl)phenoxy]-pentanoic acid.

EXAMPLE 25

(E)-6-[2,3-dichloro-4-(2-nitro-1-butenyl)phenoxy]hexanoic acid

Step A: 6-(2,3-dichloro-4-formylphenoxy)hexanoic acid

By following substantially the procedure described in Example 21, Step A, except that the ethyl 4-bromobutyrate used therein is replaced by an equimolar quantity of ethyl 6-bromohexanoate, and there is obtained 6-(2,3-dichloro-4-formylphenoxy)hexanoic acid.

Step B: (E)-6-[2,3-dichloro-4-(2-nitro-1-butenylphenoxy]hexanoic acid

By following substantially the procedure described in Example 21, Step B, except that the 4-(2,3-dichloro-4-formylphenoxy)butyric acid used therein is replaced by an equimolar amount of 6-(2,3-dichloro-4-formylphenoxy)hexanoic acid, and there is obtained (E)-6-[2,3-dichloro-4-(2-nitro-1-butenyl)phenoxy]-hexanoic acid.

EXAMPLE 26

(E)-1-[2,3-dichloro-4-(2-nitro-1-butenyl)phenoxy]cyclobutane-1-carboxylic acid

Step A: 1-(2,3-dichloro-4-formylphenoxy)cyclobutane-1-carboxylic acid

By following substantially the procedure described in Example 21, Step A except that the ethyl 4-bromobutyrate is replaced by an equimolar quantity of ethyl 1-bromocyclobutanecarboxylate, and there is obtained 1-(2,3-dichloro-4-formylphenoxy)cyclobutane-1-carboxylic acid.

Step B: (E)-1-[2,3-dichloro-4-(2-nitro-1-butenyl)phenoxy]cyclobutane-1-carboxylic acid

By following substantially the procedure described in Example 21, Step B, except that the 4-(2,3-dichloro-4-formylphenoxy)butyric acid used therein is replaced by an equimolar quantity of 1-(2,3-dichloro-4-formylphenoxy)cyclobutane-1-carboxylic acid.

EXAMPLE 27

(E)-1-[2,3-dichloro-4-(2-nitro-1-pentenyl)phenoxy]cyclobutane-1-carboxylic acid

By following substantially the same procedure described in Example 21, Step B except that the 4-(2,3-dichloro-4-formylphenoxy)butyric acid and 1-nitropropane are replaced by equimolar quantities of 1-(2,3-dichloro-4-formylphenoxy)cyclobutane-1-carboxylic acid (See Example 26, Step A) and 1-nitrobutane, and there is obtained (E)-1-[2,3-dichloro-4-(2-nitro-1-pentenyl)phenoxy]cyclobutane-1-carboxylic acid

EXAMPLE 28

(E)-4-[2,3-dichloro-4-(3-methyl-2-nitro-1-butenyl)phenoxy]butyric acid

By following substantially the procedure described in Example 21, Step B except that the 1-nitropropane used therein is replaced by and equimolar quantity of 1-nitro-2-methylpropane, and there is obtained (E)-4-[2,3-dichloro-4-(3-methyl-2-nitro-1-butenyl)phenoxy]butyric acid.

EXAMPLE 29

N-{(E)-4-[2,3-Dichloro-4-(2-nitro-1-butenyl)phenoxy]butyryl}glycine

(E)-4-[2,3-Dichloro-4-(2-nitro-1-butenyl)phenoxy]butyric acid (20.89 g, 0.06 mole) is dissolved in dry benzene (45 ml) and thionyl chloride (19.04 g, 0.12 mole) and the mixture stirred and refluxed for 75 minutes. The volatile materials are removed by evaporation at reduced pressure, and the residue, which consisted of (E)-4-[2,3-dichloro-4-(2-nitro-1-butenyl)phenoxy]butyryl chloride, is dissolved in dry tetrahydrofuran (50 ml).

To a stirring suspension of finely ground glycine (6.01 g, 0.08 mole) in tetrahydrofuran (50 ml) is added, dropwise, the solution of E-4-[2,3-dichloro-4-(2-nitro-1-butenyl)phenoxy]butyryl chloride over a period of 15 minutes. The mixture is stirred at ambient temperature for 2.25 hours and then at reflux for one hour. The mixture is cooled, filtered and washed with tetrahydrofuran. The solid is suspended in water (60 ml), filtered, washed with water and dried to give 7.45 g of N-{(E)-4-[2,3-dichloro-4-(2-nitro-1-butenyl)phenoxy]-butyryl}glycine.

EXAMPLE 30

N-{(E)-4-[2,3-Dichloro-4-(2-nitro-1-butenyl)phenoxy]butyryl}-S(+)-alanine

By carrying out the reaction essentially as described in Example 29, except that the glycine was replaced by an equimolar quantity of L-alanine, there was obtained N-{(E)-4-[2,3-dichloro-4-(2-nitro-1-butenyl)phenoxy]butyryl}-S(+)-alanine.

EXAMPLE 31

N-{(E)-4-[2,3-Dichloro-4-(2-nitro-1-butenyl)phenoxy]butyryl}-S(+)-leucine

By carrying out the reaction essentially as described in Example 29, except that the glycine is replaced by an equimolar quantity of S(+)leucine, there is obtained N-{(E)-4-[2,3-dichloro-4-(2-nitro-1-butenyl)-phenoxy]butyryl}-S(+)-leucine.

EXAMPLE 32

N-{(E)-4-[2,3-Dichloro-4-(2-nitro-1-butenyl)phenoxy]butyryl}-S(+)-serine

By carrying out the reaction essentially as described in Example 29, except that the glycine is replaced by in equimolar quantity of L-serine, there is obtained N-{(E)-4-[2,3-dichloro-4-(2-nitro-1-butenyl)phenoxy]-butyryl}-S(+)-serine.

EXAMPLE 33

N-{(E)-4-[2,3-Dichloro-4-(2-nitro-2-butenyl)phenoxy]butyryl}-S(-)-phenylalanine

By carrying out the reaction essentially as described in Example 29, except that the glycine is replaced by an equimolar quantity of L-phenylalanine, there is obtained N-{(E)-4-[2,3-dichloro-4-(2-nitro-1-butenyl)-phenoxy]butyryl}-S(-)-phenylalanine.

EXAMPLE 34

N-{(E)-4-[2,3-Dichloro-4-(2-nitro-1-pentenyl)phenoxy]butyryl}glycine

By carrying out the reaction essentially as described in Example 29, except that the (E)-4-[2,3-dichloro-4-(2-methylenebutyryl)phenoxy]butyric acid is replaced by an equimolar quantity of (E)-4-[2,3-dichloro-4-(2-nitro-1-pentenyl)phenoxy]butyric acid, there is obtained N-{(E)-(4-[2,3-dichloro-4-(2-nitro-1-pentenyl)-phenoxy]butyryl}glycine.

EXAMPLE 35

N-{(E)-1-[2,3-Dichloro-4-(2-nitro-1-butenyl)phenoxy]cyclobutane-1-carbonyl}glycine

By carrying out the reaction essentially as described in Example 29, except that the (E)-4-[2,3-dichloro-4-(2-nitro-1-butenyl)phenoxy]acetic acid is replaced by an equimolar quantity of 1-[2,3-dichloro-(2-nitro-1-butenyl]cyclobutane-1-carboxylic acid, there is obtained N-{(E)-1-[2,3-dichloro-(2-nitro-1-butenyl)phenoxy]-cyclobutane-1-carbonyl}glycine.

EXAMPLE 36

N-{(E)-3-[2,3-Dichloro-4-(2-nitro-1-butenyl)phenoxy]propionyl}glycine

By carrying out the reaction essentially as described in Example 29, except that the (E)-4-[2,3-dichloro-4-(2-nitro-1-butenyl)phenoxy]butyric acid is replaced by an equimolar quantity of (E)-3-[2,3-dichloro-(2-nitro-1-butenyl)phenoxy]propionic acid, there is obtained N-{(E)-3-[2,3-dichloro-4-(2-nitro-1-butenyl)phenoxy]-propionyl}glycine.

EXAMPLE 37

N-{(E)-5-[2,3-Dichloro-4-(2-nitro-1-butenyl)phenoxy]pentanoyl}glycine

By carrying out the reaction essentially as described in Example 29, except that the (E)-4-[2,3-dichloro-4-(2-nitro-1-butenyl)phenoxy]butyric acid is replaced by an equimolar quantity of (E)-5-[2,3-dichloro-4-(2-nitro-1-butenyl)phenoxy]pentanoic acid, there is obtained N-{(E)-5-[2,3-dichloro-4-(2-nitro-1-butenyl)-phenoxy]pentanoyl}glycine.

EXAMPLE 38

N-{(E)-5-[2,3-Dichloro-4-(2-nitro-1-butenyl)phenoxy]hexanoyl}glycine

By carrying out the reaction essentially as described in Example 29, except that the (E)-4-[2,3-dichloro-4-(2-nitro-1-butenyl)phenoxy]butyric acid is replaced by an equimolar quantity of (E)-6-[2,3-dichloro-(2-nitro-1-butenyl)phenoxy]hexanoic acid, there is obtained N-{(E)-6-[2,3-dichloro-4-(2-nitro-1-butenyl)phenoxy]-hexanoyl}glycine.

EXAMPLE 39

N-{(E)-1-[2,3-Dichloro-4-(2-nitro-1-butenyl)phenoxy]cyclobutane-1-carbonyl}-3-aminopropionic acid

By carrying out the reaction essentially as described in Example 29, except that the (E)-4-[2,3-dichloro-4-(2-nitro-1-butenyl)phenoxy)butyric acid is replaced by an equimolar quantity of (E)-1-[2,3-dichloro-4-(2-nitro-1-butenyl)phenoxy]cyclobutane-1-carboxylic acid and the glycine is replaced by an equimolar quantity of $\beta$-alanine, there is obtained N-{(E)-1-[2,3-dichloro-4-(2-nitro-1-butenyl)phenoxy]cyclobutane-1-carbonyl}-3-aminopropionic acid.

EXAMPLE 40

(E) [4-(2-Nitro-1-propenyl)phenoxy]acetic Acid Step A: Ethyl (4-formylphenoxy)acetate

p-Hydroxybenzaldehyde (122 g, 1 mole), ethyl bromoacetate (167 g, 1 mole), potassium carborate (138 g, 1 mole) and acetone (500 ml) were united, stirred and refluxed for 3 hours. The reaction mixture was poured into crushed ice (2 kg). The solid that separates was removed by filtrations, washed with water, dried and recrystallized from ethanol to give 157 g of product, m.p. 41-42 ° C.

Step B: Ethyl [4-(butyliminomethylene)phenoxy]acetate

Ethyl (4-formylphenoxy)acetate (104.1 g, 0.5 mole), butylamine (36.6 g, 0.5 mole) and benzene (400 ml) were united and refluxed in a flask equipped with a Dean-Stark water separator. After one hour, water ceased to separate. The heating was terminated after 6 hours and the mixture distilled. There was obtained 108.6 g of product boiling at 178-181 ° C at 67 Pa (0.5 mm Hg) pressure.

Step C: Ethyl [4-(nitro-1-propenyl)phenoxy]acetate

Ethyl [4-(butyliminomethylene)phenoxy]acetate (23.6 g, 0.1 mole), nitroethane (26 g, 0.35 mole) and acetic acid (75 ml) were united and heated to boiling. After standing for 30 minutes, the solution was chilled and poured with stirring into ice water (500 ml). The solid that separated upon cooling was removed by filtration, washed with water, dried and recrystallized from ethanol. The yield product was 22 g, m.p. 80-82 ° C.

Step D: (E) [4-(2-Nitro-1-propenyl)phenoxy]acetic acid

Ethyl (E) [4-(2-nitro-1-propenyl)phenoxy] acetate (10 g, 0.0378 mole), glacial acetic acid (70 ml) water (50 ml) and concentrated hydrochloric acid (2 ml) were place in a flask and stirred and refluxed for 2 hours. The mixture was chilled in ice and the precipitate that formed was removed by filtration, washed with cold ethanol and dried. Concentration of the filtrate to a volume of 50 ml and cooling yielded more product which was removed by filtration.

The combined products were recrystallized from a 50% mixture of water and ethanol to give (E)[4-(2-nitro-1-propenyl)phenoxy]acetic acid, m.p. 149-151 ° C.

EXAMPLE 41

(E) 3-Chloro-4-(2-nitro-1-butenyl)benzoic acid

Step A: 3-Chloro-4-formylbenzoic acid

3-Chloro-4-formylbenzonitrile (16.5 g, 0.1 mole) was stirred and refluxed with concentrated hydrochloric (60 ml) for 16 hours. The mixture was cooled and the solid separated by filtration, washed with water and dried. The solid was treated with an aqueous sodium bicarbonate solution and filtered. The filtrate was acidified with dilute hydrochloric acid to give the desired product which was separated by filtration, washed with water and dried to give 7.25 g of material melting at 214-215 ° C.

Step B: (E) 3-Chloro-4-(2-nitro-1-butenyl)benzoic acid

3-Chloro-4-formylbenzoic acid (5 g, 0.027 mole), butylamine (2 g, 0.027 mole) and benzene (75 ml) were refluxed for 2 hours using a flask fitted with a Dean-Stark water separator. The solvent was removed by distillation in vacuo to give 3-chloro-4-(butyliminomethylene)benzoic acid to which was added nitropropane (8.9 g, 0.1 mole) and acetic acid (21 ml). The mixture was refluxed for 10 minutes then cooled in ice. The mixture was treated with water and the solid that separated removed by filtration, washed with water, dried and recrystallized from a mixture of acetic acid and water (2:1) to give 4.7 g of the desired product, m.p. 179-180 ° C.

Analysis for $C_{11}H_{10}ClNO_4$

Calculated, C, 51.68; H, 3.94. Found: C, 51.80; H, 4.00.

EXAMPLE 42

(E)[2-Methoxy-4-(2-nitro-1-propenyl)phenoxy]acetic acid

Step A: (2-Methoxy-4-formylphenoxy)acetic acid

Vanillin (16.3 g, 0.107 mole), ethyl bromoacetate (21.6 g, 0.128 mole), potassium carbonate (17.8 g, 0.128 mole) and N,N-dimethylformamide (75 ml) were united, stirred and heated at 50-55° C for 35 minutes. Then a 10% aqueous sodium hydroxide solution (100 ml) and water (300 ml) was added and the mixture stirred on a steam bath for one hour. The solution was cooled and acidified with hydrochloric acid. The solid that separated was removed by filtration, washed with water and dried to give 20.4 g of the desired product, m.p. 177-178° C.

Step B: (E) [2-Methoxy-4-(2-nitro-1-propenyl)phenoxy]acetic acid

(2-Methoxy-4-formylphenoxy)acetic acid (10.5 g, 0.05 mole), butylamine (14.6 g, 0.2 mole) and benzene were united and refluxed in a flask equipped with a Dean-Stark water separator. The mixture was refluxed for 4 hours and then the benzene removed by distillation at reduced pressure. The residue consisted of 2-methoxy-4-(butyliminomethylene)phenoxy)acetic acid which was treated with nitropropane (17.8 g, 0.2 mole) and acetic acid (50 ml) and heated to boiling. After standing for 30 minutes the mixture was poured into crushed ice and acidified with hydrochloric acid. The solid that separated was removed by filtration, washed with water, dried and recrystallized first from a mixture of water and isopropyl alcohol (3:1) and then from a mixture of benzene and hexane (4:3) to give product melting at 116-117° C.
Anal. Calc. for $C_{13}H_{15}NO_4$: C, 55.51; H, 5.38 N, 4.95.
Found: C, 55.82; H, 5.40; N, 4.66.

EXAMPLE 43

An example of extracorporeal treatment will be considered.

0.57dm$^3$ (One pint) of blood from a homozygous patient's cells is removed and washed under sterile conditions with a 1 to 3 mM solution of the sodium salt of 4-[2,3-dichloro-4-(2-methylenebutyryl)phenoxy]-butyric acid,

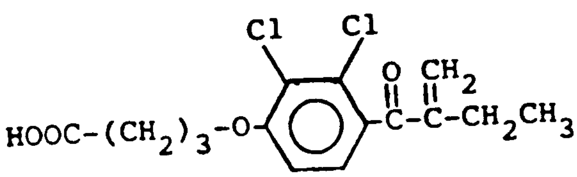

This washing is performed until the hemoglobin S is substantially completely converted to the new species having the sodium salt of the compound bound to the hemoglobin. This may be accomplished by washing the cells with about 4 to 6 liters of a 1 to 3 mM solution of the sodium salt of the compound. The reaction is monitored by the electrophoresis of a blood sample which demonstrates the percentage of conversion to the new hemoglobin derivative. The treated cells are then washed thoroughly with isotonic saline until substantially all of the excess and lightly bound drug is removed from the hemoglobin and the red cell membranes. The treated blood may then be reconstituted with plasma or nutrients or given directly to the patient.

The solubility assay ($C_{sat}$ assay) measures the ability of a compound to increase solubility of sickle hemoglobin (HbS). At low concentrations (5 mM), the sodium salt of 4-[2,3-dichloro-4-(2-methylenebutyryl)-phenoxy]butyric acid exhibited a higher solubility ratio than other antisickling agents, such as, dichlorobenzyloxyacetic acid and p-bromobenzyloxyacetic acid. The higher the ratio, the higher the activity of the compound. Chromatographic separation confirmed the fact that desired tight covalent binding between the HbS and the therapeutic agent had occurred. Tests of transport across erythrocyte membranes confirmed the ability of the agent to cross the red blood cell membrane and react with hemoglobin.

It is believed that the compound functions by covalently bonding to the hemoglobin at two locales and forms a new kind of hemoglobin which does not sickle. This is not true of clofibric acid or other phenoxy alkanoic acids.

The process may be practiced extracorporeally although other means of administering the medication such as orally may be employed. The presently preferred oral dosage is about 50 mg to 2.5 g/day.

EXAMPLE 44

| Dry filled capsules containing 150 mg of active ingredient | |
|---|---|
| | Per Capsule |
| 4-[2,3-Dichloro-4-(2-methylenebutyryl)phenoxy]butyric acid<br>Lactose<br>Magnesium stearate | 150 mg<br>447 mg<br>3 mg |
| Capsule (size no. 000) | 600 mg |

4-[2,3-Dichloro-4-(2-methylenebutyryl)phenoxy]butyric acid is reduced to a No. 60 powder and then lactose and magnesium stearate are passed through a No. 60 bolting cloth onto the powder and the combined ingredients admixed for 10 minutes and then filled into a No. 1 dry gelatin capsule.

Similar dry-filled capsules can be prepared by replacing the active ingredient of the above example by any of the other compounds of this invention.

EXAMPLE 45

Parenteral Solution of Sodium 3-[2,3-Dichloro-4-(2-methylenebutyryl)phenoxy]propionate

25 mg of 3-[2,3-dichloro-4-(2-methylenebutyryl)phenoxy]propionic acid is dissolved in 3.1 ml of 0.1 N sodium bicarbonate and sufficient isotonic buffer to make a final volume of 10 ml. The water from all sources was pyrogen-free. The solution is sterilized by filtration.

EXAMPLE 46

Parenteral Solution of Sodium 2-[2,3-Dichloro-4-(2-methylenebutyryl)phenoxy]-2-methylpropionate

250 mg of 2-[2,3-dichloro-4-(2-methylenebutyryl)phenoxy]methylpropionicacid is dissolved in 31 ml of 0.1 N sodium bicarbonate and sufficient isotonic buffer to make a final volume of 100 ml. The water from all sources was pyrogen-free. The solution is sterilized by filtration.

EXAMPLE 47

Parenteral Solution of Sodium 4-[2,3-dichloro-4-(2-methylenevaleryl)phenoxy]butyrate

250 mg of 4-[2,3-dichloro-4-(2-methylenevaleryl)phenoxy]butyric acid is dissolved in 62 ml of 0.1N sodium bicarbonate and sufficient isotonic buffer to make a final volume of 100 ml. The water from all sources was pyrogen free. The solution is sterilized by filtration.

EXAMPLE 48

Parenteral Solution of Sodium 1-(2,3-Dichloro-4-(2-methylenevaleryl)phenoxy]cyclobutane-1-carboxylate

200 mg of 1-[2,3-dichloro-4-(2-methylenevaleryl)phenoxy]cyclobutane-1-carboxylic acid is dissolved in 31 ml of 0.1N sodium bicarbonate and sufficient isotonic buffer to make a final volume of 100 ml. The water from all sources was pyrogen free. The solution is sterilized by filtration.

EXAMPLE 49

Sterile Solution of Sodium 1-[2,3-Dichloro-4-(2-methylenebutyryl)phenoxy]cyclobutane-1-carboxylate for Extracorporeal Treatment of Red Blood Cells from Patients with Sickle Cell Anemia

0.103 g of 1-[2,3-Dichloro-4-(2-methylenebutyryl)phenoxy]cyclobutane-1-carboxylic acid is dissolved 33 ml of 0.1 N sodium bicarbonate and with isotonic saline to make a final volume of 100 ml. The water from all sources was pyrogen free. The solution is sterilized by filtration. This solution is 3 mM in active antisickling agent.

EXAMPLE 50

Parenteral Solution of the Sodium salt of N-{3-[2,3-Dichloro-4-(2-methylenebutyryl)phenoxy]-propionyl}glycine

N-{3-[2,3-Dichloro-4-(2-methylenebutyryl)phenoxy]propionyl}glycine (29 mg) is dissolved in 3.1 ml of 0.1 N sodium bicarbonate and sufficient isotonic buffer to make a final volume of 10 ml. The water from all sources was pyrogen-free. The solution is sterilized by filtration.

EXAMPLE 51

Parenteral Solution of the Sodium Salt of N-{1-[2,3-Dichloro-4-(2-methylenebutyryl)phenoxy]cyclobutane-1-carbonyl}-S(+)-alanine

250 mg of N-{1-[2,3-dichloro-4-(2-methylenebutyryl)phenoxy]cyclobutane-1-carbonyl)-S(+)-alanine (1.16 g) is dissolved in 31 ml of 0.1 N sodium bicarbonate and sufficient isotonic buffer to make a final volume of 100 ml. The water from all sources was pyrogen-free. The solution is sterilized by filtration.

EXAMPLE 52

Parenteral Solution of the Sodium Salt of N-{1-[2,3-dichloro-4-(2-methylenebutyryl)phenoxy]cyclobutane-1-carbonyl}-3-aminopropionic acid

N-{1-[2,3-Dichloro-4-(2-methylenebutyryl)phenoxy]cyclobutane-1-carbonyl}-3-aminopropionic acid (300 mg) is dissolved in 62 ml of 0.1N sodium bicarbonate and sufficient isotonic buffer to make a final volume of 100 ml. The water from all sources was pyrogen free. The solution is sterilized by filtration.

EXAMPLE 53

Parenteral Solution of the Sodium Salt of N-{1-[2,3-Dichloro-4-(2-methylenevaleryl)phenoxy]cyclobutane-1-carbonyl}glycine

N-{1-[2,3-Dichloro-4-(2-methylenevaleryl)phenoxy]cyclobutane-1-carbonyl}glycine (200 mg) is dissolved in 31 ml of 0.1N sodium bicarbonate and sufficient water to make a final volume of 100 ml. The water from all sources was pyrogen free. The solution is sterilized by filtration.

EXAMPLE 54

Sterile Solution of the Sodium Salt of N-{1-[2,3-Dichloro-4-(2-methylenebutyryl)phenoxy]cyclobutane-1-carbonyl)glycine for Extracorporeal Treatment of Red Blood Cells from Patients with Sickle Cell Anemia

N-{1-[2,3-Dichloro-4-(2-methylenebutyryl)phenoxy]cyclobutane-1-carbonyl}glycine (120 mg) is dissolved 33 ml of 0.1 N sodium bicarbonate and sufficient water to make a final volume of 100 ml. The water from all sources was pyrogen free. The solution is sterilized by filtration. This solution is 3 mM in active antisickling agent.

EXAMPLE 55

Parenteral Solution of Sodium (E)-4-[2,3-Dichloro-4-(2-nitro-1-butenyl)phenoxy]butyrate

250 mg of (E)-4-[2,3-dichloro-4-(2-nitro-1-butenyl)phenoxy]butyric acid is dissolved in 3.1 ml of 0.1 N sodium bicarbonate and sufficient isotonic buffer to make a final volume of 10 ml. The water from all sources was pyrogen-free. The solution is sterilized by filtration.

EXAMPLE 56

Parenteral Solution of Sodium (E)-1-[2,3-Dichloro-4-(2-nitro-1-butenyl)phenoxy]cyclobutane-1-carboxylate

250 mg of (E)-1-[2,3-dichloro-4-(2-nitro-1-butenyl)phenoxy]cyclobutane-1-carboxylic acid is dissolved in 31 ml of 0.1 N sodium bicarbonate and sufficient isotonic buffer to make a final volume of 100 ml. The water from all sources was pyrogen-free. The solution is sterilized by filtration.

EXAMPLE 57

Parenteral Solution of Sodium (E) 3-Chloro-4-(2-nitro-1-butenyl)benzoic acid

One gram of (E) 3-chloro-4-(2-nitro-1-butenyl)benzoic acid is dissolved in 40 ml of 0.1N sodium bicarbonate and sufficient isotonic buffer to make a final volume of 100 ml. The water from all sources was pyrogen-free. The solution is sterilized by filtration.

EXAMPLE 58

Parenteral Solution of Sodium N- (E)-4-[2,3-dichloro-4-(2-nitro-1-butenyl)phenoxy]butyryl glycine

Two grams of N- (E)-4-[2,3-dichloro-4-(2-nitro-1-butenyl)phenoxy]butyryl glycine is dissolved in 62 ml of 0.1N sodium bicarbonate and sufficient isotonic buffer to make a final volume of 100 ml. The water from all sources was pyrogen free. The solution is sterilized by filtration.

EXAMPLE 59

Parenteral Solution of Sodium N- (E)-1-[2,3-Dichloro-4-(2-nitro-1-butenyl)phenoxy]cyclobutane-1-carbonyl glycine

0.103g of N- (E)-1-[2,3-dichloro-4-(2-nitro-1-butenyl)phenoxy]cyclobutane-1-carbonyl glycine is dissolved in 31 ml of 0.1N sodium bicarbonate and sufficient isotonic buffer to make a final volume of 100 ml. The water from all sources was pyrogen free. The solution is sterilized by filtration.

EXAMPLE 60

Sterile Solution of Sodium (E)-4-[2,3-Dichloro-4-(2-nitro-1-butenyl)phenoxy]butyrate for Extracorporeal Treatment of Red Blood Cells from Patients with Sickle Cell Anemia

1.0297 g of (E)-4-[2,3-Dichloro-4-(2-nitro-1-butenyl)phenoxy)butyric acid is dissolved 33 ml of 0.1 N sodium bicarbonate and sufficient isotonic buffer to make a final volume of 100 ml. The water from all sources was pyrogen free. The solution is sterilized by filtration. This solution is 3 mM in active antisickling agent.

EXAMPLE 61

Sterile Solution of Sodium (E) [2-methoxy-4-(2-nitro-1-propenyl)phenoxy]acetate for Extracorporeal Treatment of Red Blood Cells for Patients with Sickle Cell Anemia

One gram of (E) [2-methoxy-4-(2-nitro-1-propenyl)phenoxy]acetic acid is dissolved in 35.6 ml of 0.1N sodium bicarbonate and sufficient isotonic buffer to make a final volume of 100 ml. The water from all souces was pyrogen free. The solution is sterilized by filtration. This solution is 3.56 mM in active antisickling agent.

It will be appreciated that the present invention provides a method for treatment of sickle cell anemia patients so as to resist undesired sickle cell anemia crisis. The method may advantageously be employed as a prophylactic.

Whereas particular embodiments of the invention have been described above for purposes of illustration, it will be evident to those skilled in the art that numerous variations of the details may be made without departing from the invention as defined in the appended claims.

EXAMPLE 62

In order to confirm the effectiveness of these compounds in reducing the effect of sickle cell anemia, tests were performed. The materials were tested according to the assay ($C_{sat}$) developed by Hofrichter et al. (J. Hofrichter, P. D. Ross and W. A. Eaton, Proc. Natl. Acad. Sci., U.S.A. 73, 30-35, 1976). This assay involves deoxygenation of concentrated sickle hemoglobin with dithionite in the presence of different concentrations of the drugs being tested. Samples are then sealed in quartz epr tubes under anaerobic conditions and spun at about 150,000 g for about 2-1/2 hours at about 35 °C in an ultracentrifuge. This procedure pellets the polymerized HbS (sickle hemoglobin) to the bottom of the tubes and the supernatant (soluble HbS) is measured in the laboratory as the cyanmethemoglobulin derivative. The more active the compound the greater the solubility of HbS and the smaller the pellet size. Activity is reported as a ratio of the HbS solubility with the particular drug to HbS solubility with no drug, i.e. control. The higher the ratio the greater degree of activity of the drug. The numbers in parenthesis are the percent increase in solubility of

HbS; again, the higher the number, the greater the activity. The results of representative compounds from the Examples are shown in Table I as typical of the compounds of the invention.

Table I

| Example Number of the Compound Tested | Ratio of the HbS Solubility with the Drug Compared to HbS Solubility Without Drug (% increase in Solubility) at the Concentrations Indicated | | | |
|---|---|---|---|---|
| | 3 mM | 5 mM | 7 mM | 9 mM |
| 1 | 1.112(26) | 1.145(33) | 1.188(43) | 1.302(69) |
| 2 | 1.092(22) | 1.112(27) | 1.157(37) | 1.196(46) |
| 3 | 1.075(18) | 1.159(37) | 1.197(46) | 1.219(51) |
| 4 | 1.081(21) | 1.129(34) | 1.200(52) | 1.308(80) |
| 5 | 1.063(13) | 1.086(18) | 1.125(26) | 1.164(35) |
| 6 | 1.080(19) | 1.126(30) | 1.147(34) | 1.202(48) |
| 7 | 1.057(14) | 1.122(30) | 1.104(26) | 1.156(39) |
| 8 | 1.050(11) | 1.094(21) | 1.183(41) | 1.237(53) |
| 9 | 1.109(27) | 1.145(36) | 1.222(56) | 1.327(82) |
| 21 | 1.037(8) | 1.063(14) | 1.069(16) | 1.093(21) |
| 40 | 1.058(13) | 1.080(19) | 1.080(19) | 1.097(28) |
| 41 | 1.052(13) | 1.100(26) | 1.106(27) | 1.150(39) |
| 42 | 1.092(23) | 1.121(30) | 1.153(37) | 1.226(55) |
| | 1.079(19) | 1.136(33) | 1.165(40) | 1.188(45) |

## Claims

1. A compound and pharmaceutically acceptable salts of the general formula (I):

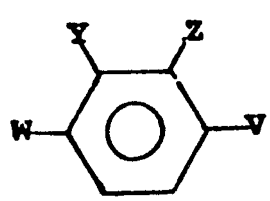

(I)

wherein V is selected from:

(a)

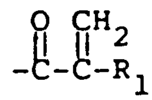

in which

$R_1$ is ethyl, propyl or isopropyl;

(b)

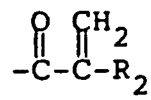

in which

$R_2$ is H or lower alkyl;

(c)

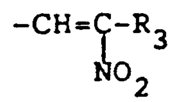

in which

$R_3$ is lower alkyl;

(d)

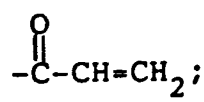

W is selected from:

(a)

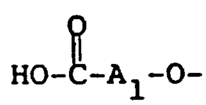

in which

$A_1$ is $(CH_2)n_1$, $-C(CH_2)_3$ and

$n_1 = 2,4$, or 5;

(b)

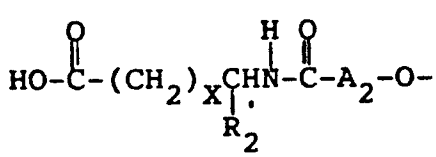

in which

$R_2'$ is H, lower alkyl, benzyl, $CH_2OH$;

$A_2$ is $(CH_2)n_2$ $-C(CH_3)_2$, $-C(CH_2)_3$-and $n_2$ = 1 to 5;

X is 0 or 1;

(c)

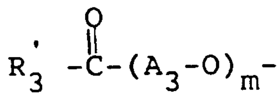

in which

$R_3'$ is HO- or

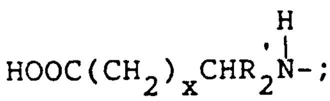

$A_3$ is $-(CH_2)n_3$ or $-C(CH_2)_3$,- and
$n_3$ = 1 to 5;
m is 0 or 1;

(d)

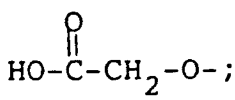

and
Y is H, Cl or $OCH_3$;
Z is H or Cl;

provided that

(i) when W is

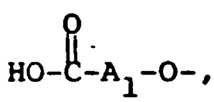

V is

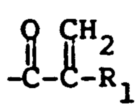

and Y and Z are Cl;

(ii) when W is

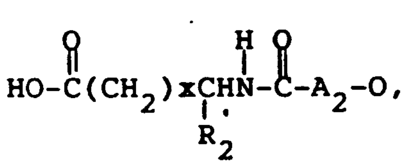

V is

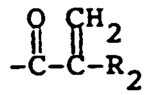

Y and Z are Cl, with the proviso that when $R_2$ is $C_2H_5$, $R_2'$ is H and X is O, $n_2$ is 2 to 5; provided that when $R_2$ is isopropyl, $R_2'$ is $CH_3$ and X is O, $n_2$ is 2 to 5;

(iii) when W is

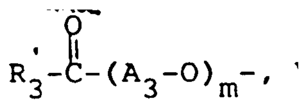

V is

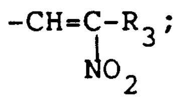

with the proviso that when $R_3'$ is OH, Y is H or Cl and m is 1, $n_3 = 4$ or 5;

(iv) when W is

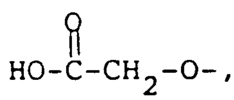

V is

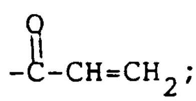

and Y and Z are Cl.

**2.** A compound, and pharmaceutically acceptable salts according to Claim 1, selected from the group consisting of:

a. 3-[2,3-dichloro-4-(2-methylenebutyryl)phenoxy]propionic acid,
b. 5-[2,3-dichloro-4-(2-methylenebutyryl)phenoxy]valeric acid,
c. 6-[2,3-dichloro-4-(2-methylenebutyryl)phenoxy]hexanoic acid,
d. 1-[2,3-dichloro-4-(2-methylenebutyryl)phenoxy]cyclobutane-1-carboxylic acid,
e. 1-[2,3-dichloro-4-(2-methylenevaleryl)phenoxy]cyclobutane-1-carboxylic acid.

**3.** A compound, and pharmaceutically acceptable salts according to Claim 1, selected from the group consisting of:

a. N-{[2,3-dichloro-4-(2-methylenebutyryl)phenoxy]acetyl}-S(+)-alanine,
b. N-{[2,3-dichloro-4-(2-methylenebutyryl)phenoxy]acetyl}-S(+)-serine,
c. N-{1-[2,3-dichloro-4-(2-methylenebutyryl)phenoxy]cyclobutane-1-carbonyl}glycine,
d. N-{1-[2,3-dichloro-4-(2-methylenevaleryl)phenoxy]cyclobutane-1-carbonyl}glycine,
e. N-{[2,3-dichloro-4-(2-methylenebutyryl)phenoxy]acetyl}-S(-)-phenylalanine,
f. N-{4-[2,3-dichloro-4-(2-methylenebutyryl)phenoxy]butyryl}glycine,
g. N-{3-[2,3-dichloro-4-(2-methylenebutyryl)phenoxy]propionyl}glycine,
h. N-{1-[2,3-dichloro-4-(2-methylenebutyryl)phenoxy]cyclobutane-1-carbonyl)-3-aminopropionic acid,
i. N-[(2,3-dichloro-4-acryloylphenoxy)acetyl]glycine,
j. N-{1-[2,3-dichloro-4-(2-methylenebutyryl)phenoxy]cyclobutane-1-carbonyl-S(+)-alanine,

k. N-{[2,3-dichloro-4-(2-methylenebutyryl)phenoxy]acetyl}-S(+)-leucine.

**4.** A compound and pharmaceutically acceptable salts of Claim 1 selected from a group consisting of the following compounds wherein:

a. $R'_3$ is OH, $A_3$ is $(CH_2)_4$, $R_3$ is $C_2H_5$, Y and Z are Cl and m is 1,
b. $R'_3$ is OH, $A_3$ is $(CH_2)_5$, $R_3$ is $C_2H_5$, Y and Z are Cl and m is 1,
c. $R'_3$ is OH, $A_3$ is

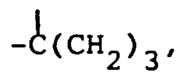

$R_3$ is $C_2H_5$, Y and Z are Cl and m is 1,
d. $R'_3$ is OH, $A_3$ is $-C(CH_2)_3$, $R_3$ is $CH_2CH_2CH_3$, Y and Z are Cl m is 1,
e. $R'_3$ is $HOOCCH_2NH$, $A_3$ is $-(CH_2)_3-$, $R_3$ is $C_2H_5$, Y and Z are Cl and m is 1,
f. $R'_3$ is S(+)-$HOOCCH(CH_3)NH$-, $A_3$ is $(CH_2)_3$, $R_3$ is $C_2H_5$, Y and Z are Cl and m is 1,
g. $R'_3$ is S(+)-$HOOCCH[CH_2CH(CH_3)_2]NH$ $A_3$ is $(CH_2)_3$, $R_3$ is $C_2H_5$, Y and Z are Cl and m is 1,
h. $R'_3$ is S(+)-$HOOCCH(CH_2OH)NH$-, $A_3$ is $(CH_2)_3$, $R_3$ is $C_2H_5$, Y and Z are Cl and m is 1,
i. $R'_3$ is S(-)-$HOOCCH(CH_2C_6H_5)NH$-$A_3$ is $(CH_2)_3$, $R_3$ is $C_2H_5$, Y and Z are Cl and m is l,
j. $R'_3$ is $HOOCCH_2NH$-, $A_3$ is $(CH_2)_3$, $R_3$ is $CH_2CH_2CH_3$, Y and Z are Cl and m is l,
k. $R'_3$ is $HOOCCH_2NH$-, $A_3$ is $-C(CH_2)_3$, $R_3$ is $C_2H_5$, Y and Z Cl and m is l,
l. $R'_3$ is $HOOCCH_2NH$, $A_3$ is $(CH_2)_2$, $R_3$ is $C_2H_5$, Y and Z are Cl and m is l,
m. $R'_3$ is $HOOCCH_2NH$-, $A_3$ is $(CH_2)_4$, $R_3$ is $C_2H_5$, Y and Z are Cl and m is l,
n. $R'_3$ is $HOOCCH_2NH$-, $A_3$ is $(CH_2)_3$, $R_3$ is $C_2H_5$, Y and Z are Cl and m is l,
o. $R'_3$ is $HOOCCH_2CH_2NH$, $A_3$ is $-C(CH_2)_3$, $R_3$ is $C_2H_5$, Y and Z are Cl and m is l,
p. $R'_3$ is OH, Y is $OCH_3$, Z is H, $R_3$ is $CH_3$, $A_3$ is $CH_2$ and m is l,
q. $R'_3$ is OH, Y is H, Z is Cl, $R_3$ is $C_2H_5$ and m is O.

**5.** The use of an antisickling agent the preparation of a medicament useful for the treatment of sickle cell anemia wherein the antisickling agent is a compound and pharmaceutically acceptable salts thereof, selected from the following general formulae:

a.

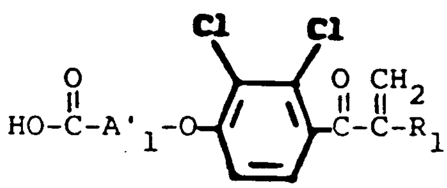

wherein $R_1$ is ethyl, propyl or isopropyl and

$A'_1$ is $-(CH_2)n'_1$ $-C(CH_3)_2-$ or

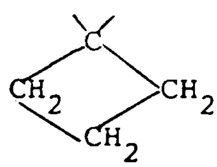

and $n'_1 = 2$-5; or

b.

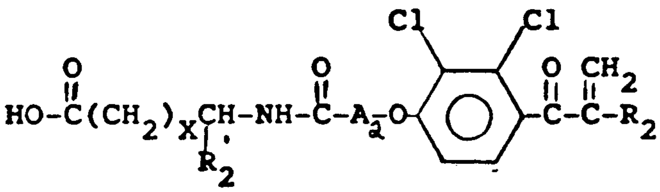

wherein:

| | |
|---|---|
| $R_2$ = | H or lower alkyl; |
| $R_2'$ = | H, lower alkyl, benzyl, $CH_2OH$; |
| $A_2$ = | $(CH_2)n_2$, $-C(CH_3)_2$, or $C(CH_2)_3$; |
| $n_2$ = | 1 to 5; and |
| X = | 0 or 1; or |

c.

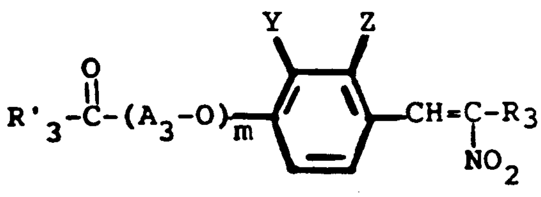

wherein

| | |
|---|---|
| $A_3$ = | lower alkyl; |
| $R_3$ = | HO or $HOOC(CH_2)_xCHR_2'$ NH-; |
| $R_2'$ = | H, lower alkyl, benzyl or $-CH_2OH$; |
| x = | 0 or 1; |
| $A_3$ = | $-(CH_2)n_3-$ or $-C(CH_2)_3$; |
| $n_3$ = | 1 to 5; and |
| Y = | H, Cl or $OCH_3$, |
| Z = | H or Cl, |
| m = | 0 or 1. |

d.

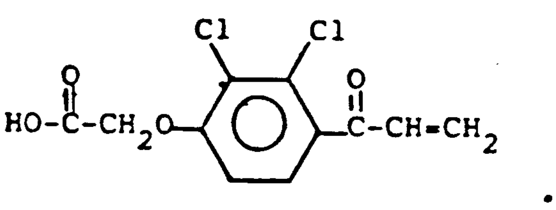

6. An antisickling pharmaceutical composition comprising a pharmaceutical carrier and a nontoxic therapeutically active amount of an antisickling agent wherein the antisickling agent is selected from:
   a. 3-[2,3-dichloro-4-(2-methylenebutyryl)phenoxy]propionic acid,
   b. 4-[2,3-dichloro-4-(2-methylenebutyryl)phenoxy]butyric acid,
   c. 5-[2,3-dichloro-4-(2-methylenebutyryl)phenoxy]valeric acid,
   d. 6-[2,3-dichloro-4-(2-methylenebutyryl)phenoxy]hexanoic acid,
   e. 2-[2,3-dichloro-4-(2-methylenebutyryl)phenoxy]-2-methylpropionic acid,
   f. 1-[2,3-dichloro-4-(2-methylenebutyryl)phenoxy]cyclobutane-1-carboxylic acid,
   g. 1-[2,3-dichloro-4-(2-methylenevaleryl)phenoxy]cyclobutane-1-carboxylic acid
   h. 4-[2,3-dichloro-4-(2-methylenevaleryl)phenoxy]butyric acid.

**7.** An antisickling pharmaceutical composition comprising a pharmaceutical carrier and a nontoxic therapeutically active amount of an antisickling agent wherein the antisickling agent is selected from:

a. N-{[[2,3-dichloro-4-(2-methylene butyryl)phenoxy]acetyl}-S(+)-alanine,
b. N-{[2,3-dichloro-4-(2-methylene butyryl)phenoxy]acetyl}-S(+)-serine,
c. N-{1-[2,3-dichloro-4-(2-methylene butyryl)phenoxy]cyclobutane-1-carbonyl}glycine,
d. N-{1-[2,3-dichloro-4-(2-methylenevaleryl)phenoxy]cyclobutane-1-carbonyl}glycine,
e. N-{[2,3-dichloro-4-(2-methylenebutyryl)phenoxy]acetyl}-S(-)-phenylalanine,
f. N-{4-[2,3-dichloro-4-(2-methylenebutyryl)phenoxy]butyryl}glycine,
g. N-{3-[2,3-dichloro-4-(2-methylenebutyryl)phenoxy]propionyl}glycine,
h. N-{1-[2,3-dichloro-4-(2-methylenebutyryl)phenoxy]cyclobutane-1-carbonyl)-3-aminopropionic acid,
i. N-[(2,3-dichloro-4-acryloylphenoxy)acetyl]glycine,
j. N-{1-[2,3-dichloro-4-(2-methylenebutyryl)phenoxy]cyclobutane-1-carbonyl}-S(+)-alanine,
k. N-{[2,3-dichloro-4-(2-methylenebutyryl)phenoxy]acetyl}S(+)-leucine.

**8.** An antisickling pharmaceutical composition comprising a pharmaceutical carrier and a nontoxic therapeutically active amount of an antisickling agent wherein the antisickling agent has the formula c. in claim 5 wherein:

a. R'$_3$ is OH, Y and Z are H, $A_3$ is $CH_2$, $R_3$ is $CH_3$ and m is l,
b. R'$_3$ is OH, Y and Z are Cl, $A_3$ is $(CH_2)_3$, $R_3$ is $C_2H_5$ and m is l.

## Revendications

**1.** Composé et ses sels pharmaceutiquement acceptables de formule générale (I) :

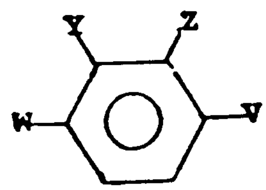

(I)

dans laquelle V est choisi parmi :

(a)

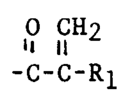

où $R_1$ est un groupe éthyle, propyle ou isopropyle ;

(b)

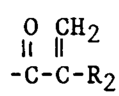

où $R_2$ est H ou un groupe alkyle inférieur ;

(c)

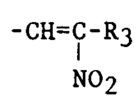

où $R_3$ est un groupe alkyle inférieur;

(d)

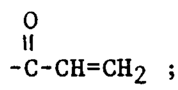

W est choisi parmi :

(a)

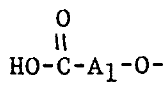

où $A_1$ est $(CH_2)n_1$, $-C(CH_2)_3$ et $n_1$ = 2, 4 ou 5 ;

(b)

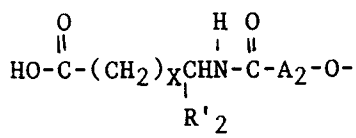

où $R'_2$ est H, un groupe alkyle inférieur, benzyle, $CH_2OH$ ; $A_2$ est $(CH_2)n_2$, $-C(CH_3)_2$, $-C(CH_2)_3$- et $n_2$ = 1 à 5 ; X vaut 0 ou 1 ;

(c)

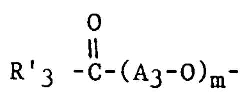

où $R'_3$ est HO- ou

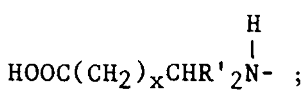

$A_3$ est $-(CH_2)n_3$ ou $-C(CH_2)_3$'- et $n_3$ = 1 à 5 ; m vaut 0 ou 1 ;

(d)

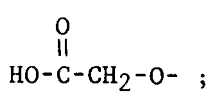

et

Y est H, Cl ou $OCH_3$ ;

Z est H ou Cl ;

à la condition que

(i) lorsque W est

$$HO-\overset{\overset{O}{\parallel}}{C}-A_1-O,$$

V est

$$-\overset{\overset{O}{\parallel}}{C}-\overset{\overset{CH_2}{\parallel}}{C}-R_1$$

et Y et Z sont Cl ;
(ii) lorsque W est

$$HO-\overset{\overset{O}{\parallel}}{C}(CH_2)x\underset{\underset{R'_2}{|}}{C}H\overset{\overset{H}{|}}{N}-\overset{\overset{O}{\parallel}}{C}-A_2-O,$$

V est

$$-\overset{\overset{O}{\parallel}}{C}-\overset{\overset{CH_2}{\parallel}}{C}-R_2$$

Y et Z sont Cl, à la condition que quand $R_2$ est $C_2H_5$, $R'_2$ est H et X vaut 0, $n_2$ vaut 2 à 5 ; à condition que lorsque $R_2$ est le groupe isopropyle, $R'_2$ est $CH_3$ et X est 0, $n_2$ vaut 2 à 5 ;
(iii) Lorsque W est

$$R'_3-\overset{\overset{O}{\parallel}}{C}-(A_3-O)_m,$$

V est

$$-CH=\underset{\underset{NO_2}{|}}{C}-R_3 \;\;;$$

à la condition que lorsque $R'_3$ est OH, Y est H ou Cl et m vaut 1, $n_3$ = 4 ou 5 ;

(iv) lorsque W est

$$HO-\overset{O}{\overset{\|}{C}}-CH_2-O-,$$

V est

$$-\overset{O}{\overset{\|}{C}}-CH=CH_2 \ ;$$

et Y et Z sont Cl.

**2.** Composé ,et ses sels pharmaceutiquement acceptables selon la revendication 1, choisi dans le groupe comprenant :

a. l'acide 3-(2,3-dichloro-4-(2-méthylènebutyryl)phénoxy)propionique
b. l'acide 5-(2,3-dichloro-4-(2-méthylènebutyryl)phénoxy)valérique
c. l'acide 6-(2,3-dichloro-4-(2-méthylènebutyryl)phénoxy)hexanoïque
d. l'acide 1-(2,3-dichloro-4-(2 -méthylènebutyryl)phénoxy)cyclobutane-1-carboxylique
e. l'acide 1-(2,3-dichloro-4-(2-méthylènevaléryl)phénoxy)cyclobutane-1-carboxylique.

**3.** Composé, et ses sels pharmaceutiquement acceptables selon la revendication 1, choisi dans le groupe comprenant :

a. la N-((2,3-dichloro-4-(2-méthylènebutyryl)phénoxy)acétyl)-S(+)-alanine
b. la N-((2,3-dichloro-4-(2-méthylènebutyryl)phénoxy)acétyl)-S(+)-sérine
c. la N-(1-(2,3-dichloro-4-(2-méthylènebutyryl)phénoxy)cyclobutane-1-carbonyl)glycine
d. la la N-(1-(2,3-dichloro-4-(2-méthylènevaléryl)phénoxy)cyclobutane-1-carbonyl)glycine
e. la N-((2,3-dichloro-4-(2-méthylènebutyryl)phénoxy)acétyl)-S(-)-phénylalanine
f. la N-(4-(2,3-dichloro-4-(2-méthylènebutyryl)phénoxy)butyryl)glycine
g. la N-(3-(2,3-dichloro-4-(2-méthylènebutyryl)phénoxy)propionyl)glycine
h. l'acide N-(1-(2,3-dichloro-4-(2-méthylènebutyryl)phénoxy)cyclobutane-1-carbonyl)-3-aminopropionique
i. la N-((2,3-dichloro-4-acryloylphénoxy)acétyl)glycine
j. la N-(1-(2,3-dichloro-4-(2-méthylènebutyryl)phénoxy)cyclobutane-1-carbonyl-S(+)-alanine
k. la N-((2,3-dichloro-4-(2-méthylènebutyryl)phénoxy)acétyl)-S(+)-leucine

**4.** Composé et ses sels pharmaceutiquement acceptables selon la revendication 1, choisis dans un groupe comprenant les composés suivants dans lesquels :

a. $R'_3$ est OH, $A_3$ est $(CH_2)_4$, $R_3$ est $C_2H_5$, Y et Z sont Cl et m vaut 1 ;
b. $R'_3$ est OH, $A_3$ est $(CH_2)_5$, $R_3$ est $C_2H_5$, Y et Z sont Cl et m vaut 1,
c. $R'_3$ est OH, $A_3$ est

$$-\overset{|}{C}-(CH_2)_3,$$

$R_3$ est $C_2H_5$, X et Z sont Cl et m vaut 1,
d. $R'_3$ est OH, $A_3$ est $-C(CH_2)_3$, $R_3$ est $CH_2CH_2CH_3$, Y et Z sont Cl, m vaut 1,
e. $R'_3$ est $HOOCCH_2NH$, $A_3$ est $-(CH_2)_3-$, $R_3$ est $C_2H_5$, Y et Z sont Cl et m vaut 1,
f. $R'_3$ est s(+)-$HOOCCH(CH_3)NH$-, $A_3$ est $(CH_2)_3$, $R_3$ est $C_2H_5$, Y et Z sont Cl et m vaut 1,
g. $R'_3$ est S(+)-$HOOCCH(CH_2CH(CH_3)_2)NH$, $A_3$ est $(CH_2)_3$, $R_3$ est $C_2H_5$, Y et Z sont Cl et m vaut 1,
h. $R'_3$ est S(+)-$HOOCCH(CH_2OH)NH$-, $A_3$ est $(CH_2)_3$, $R_3$ est $C_2H_5$, Y et Z sont Cl et m vaut 1,
i. $R'_3$ est S(-)-$HOOCCH(CH_2C_6H_5)NH$-, $A_3$ est $(CH_2)_3$, $R_3$ est $C_2H_5$, Y et Z sont Cl et m vaut 1,
j. $R'_3$ est $HOOCCH_2NH$-, $A_3$ est $(CH_2)_3$, $R_3$ est $CH_2CH_2CH_3$, Y et Z sont Cl et m vaut 1,
k. $R'_3$ est $HOOCCH_2NH$-, $A_3$ est $-C(CH_2)_3$, $R_3$ est $C_2H_5$, Y et Z sont Cl et m vaut 1,
l. $R'_3$ est $HOOCCH_2NH$, $A_3$ est $(CH_2)_2$, $R_3$ est $C_2H_5$, Y et Z sont Cl et m vaut 1,
m. $R'_3$ est $HOOCCH_2NH$-, $A_3$ est $(CH_2)_4$, $R_3$ est $C_2H_5$, Y et Z sont Cl et m vaut 1,

n. $R'_3$ est $HOOCCH_2NH$-, $A_3$ est $(CH_2)_3$, $R_3$ est $C_2H_5$, Y et Z sont Cl et m vaut 1,
o. $R'_3$ est $HOOCCH_2CH_2NH$, $A_3$ est $-C(CH_2)_3$, $R_3$ est $C_2H_5$, Y et Z sont Cl et m vaut 1,
p. $R'_3$ est OH, Y est $OCH_3$, Z est H, $R_3$ est $CH_3$, $A_3$ est $CH_2$ et m vaut 1,
q. $R'_3$ est OH, Y est H, Z est Cl, $R_3$ est $C_2H_5$ et m vaut 0.

**5.** Utilisation d'un agent anti-drépanocytaire pour la préparation d'un médicament utile pour le traitement de l'anémie drépanocytaire, dans lequel l'agent anti-drépanocytaire est un composé et ses sels pharmaceutiquement acceptables choisis parmi les formules générales suivantes :

a.

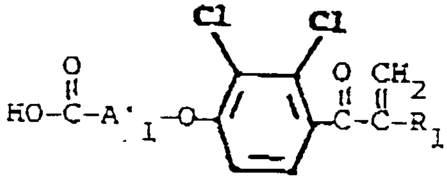

dans laquelle $R_1$ est un groupe éthyle, propyle ou isopropyle, et
$A'_1$ est $-(CH_2)n'_1$, $-C(CH_3)_2-$ ou

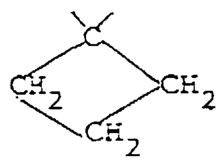

et $n'_1$ = 2 - 5 ; ou

b.

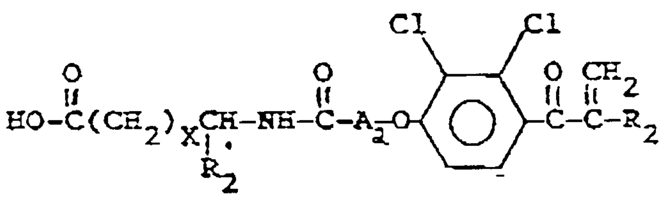

dans laquelle :

| | | |
|---|---|---|
| $R_2$ | = | H ou un groupe alkyle inférieur ; |
| $R'_2$ | = | H, un groupe alkyle inférieur, benzyle ou $CH_2OH$; |
| $A_2$ | = | $(CH_2)n_2$, $-C(CH_3)_2$, ou $C(CH_2)_3$; |
| $n_2$ | = | 1 à 5 ; et |
| X | = | 0 ou 1 ; ou |

c.

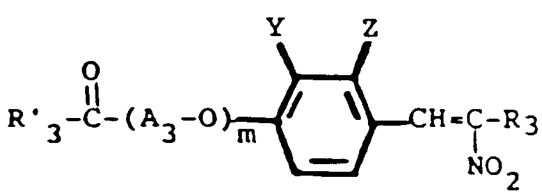

dans laquelle

$R_3$ = un groupe alkyle inférieur;
$R'_3$ = HO ou $HOOC(CH_2)_xCHR'_2NH$-;
$R'_2$ = H ou un groupe alkyle inférieur, benzyle ou $-CH_2OH$;
x = 0 ou 1;
$A_3$ = $-(CH_2)n_3$- ou $-C(CH_2)_3$;
$n_3$ = 1 à 5; et
Y = H, Cl ou $OCH_3$,
Z = H ou Cl,
m = 0 ou 1.

d.

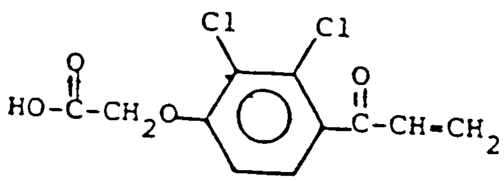

6. Composition pharmaceutique anti-dépranocytaire qui comprend un véhicule pharmaceutique et une quantité non toxique, thérapeutiquement active, d'un agent anti-drépanocytaire, dans laquelle l'agent anti-drépanocytaire est choisi parmi :
   a. l'acide 3-(2,3-dichloro-4-(2-méthylènebutyryl)phénoxy)propionique
   b. l'acide 4-(2,3-dichloro-4-(2-méthylènebutyryl)phénoxy)butyrique
   c. l'acide 5-(2,3-dichloro-4-(2-méthylènebutyryl)phénoxy)valérique
   d. l'acide 6-(2,3-dichloro-4-(2-méthylènebutyryl)phénoxy)hexanoïque
   e. l'acide 2-(2,3-dichloro-4-(2-méthylènebutyryl)phénoxy)-2-méthylpropionique
   f. l'acide 1-(2,3-dichloro-4-(2-méthylènebutyryl)phénoxy)cyclobutane-1-carboxylique
   g. l'acide 1-(2,3-dichloro-4-(2-méthylènevaléryl)phénoxy)cyclobutane-1-carboxylique
   h. l'acide 4-(2,3-dichloro-4-(2-méthylènevaléryl)phénoxy)butyrique

7. Composition pharmaceutique anti-drépanocytaire qui comprend un véhicule pharmaceutique et une quantité non toxique, thérapeutiquement active, d'un agent anti-drépanocytaire, dans laquelle l'agent anti-drépanocytaire est choisi parmi :
   a. la N-(((2,3-dichloro-4-(2-méthylènebutyryl)phénoxy)acétyl)-S(+)-alanine
   b. la N-((2,3-dichloro-4-(2-méthylènebutyryl)phénoxy)acétyl)-S(+)-sérine
   c. la N-(1-(2,3-dichloro-4-(2-méthylènebutyryl)phénoxy)cyclobutane-1-carbonyl)glycine
   d. la N-(1-(2,3-dichloro-4-(2-méthylènevaléryl)phénoxy)cyclobutane-1-carbonyl)glycine
   e. la N-((2,3-dichloro-4-(2-méthylènebutyryl)phénoxy)acétyl)-S(-)-phénylalanine
   f. la N-(4-(2,3-dichloro-4-(2-méthylènebutyryl)phénoxy)butyryl)glycine
   g. la N-(2-(2,3-dichloro-4-(2-méthylènebutyryl)phénoxy)propionyl)glycine
   h. l'acide N-(1-(2,3-dichloro-4-(2-méthylènebutyryl)phénoxy)cyclobutane-1-carbonyl)-3-aminopropionique
   i. la N-((2,3-dichloro-4-acryloylphénoxy)acétyl)glycine
   j. la N-(1-(2,3-dichloro-4-(2-méthylènebutyryl)phénoxy)cyclobutane-1-carbonyl)-S(+)-alanine
   k. la N-((2,3-dichloro-4-(2-méthylènebutyryl)phénoxy)acétyl)S(+)-leucine

8. Composition pharmaceutique anti-drépanocytaire qui comprend un véhicule pharmaceutique et une quantité non toxique, thérapeutiquement active d'un agent anti-drépanocytaire, dans laquelle l'agent dépranocytaire possède la formule c dans la revendication 5 dans laquelle
   a. $R'_3$ est OH, Y et Z sont H, $A_3$ est $CH_2$, $R_3$ est $CH_3$ et m vaut 1,
   b. $R'_3$ est OH, Y et Z sont Cl, $A_3$ est $(CH_2)_3$, $R_3$ est $C_2H_5$ et m vaut 1.

## Patentansprüche

**1.** Verbindung und pharmazeutisch annehmbare Salze der allgemeinen Formel (I)

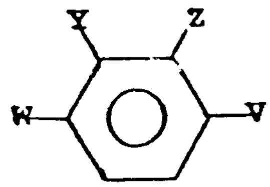

(I)

worin V ausgewählt ist aus:

(a)

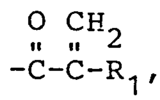

worin $R_1$ Ethyl, Propyl oder Isopropyl ist;

(b)

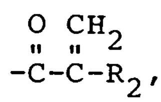

worin $R_2$ H oder Niederalkyl ist;

(c)

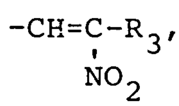

worin $R_3$ Niederalkyl ist;

(d)

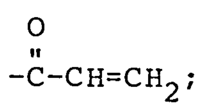

W ausgewählt ist aus:

(a)

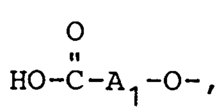

worin $A_1$ $(CH_2)n_1$, $-C(CH_2)_3$ ist und $n_1$ = 2,4 oder 5 ist;

(b)

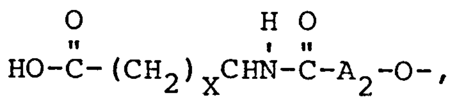

worin $R_2'$ H, Niederalkyl, Benzyl, $CH_2OH$ ist;
$A_2$ $(CH_2)n_2$, $-C(CH_3)_2$, $-C(CH_2)_3-$ ist und
$n_2$ 1 bis 5 ist;
X 0 oder 1 ist;
(c)

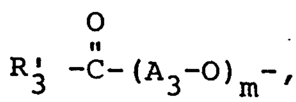

worin $R_3'$ HO- oder

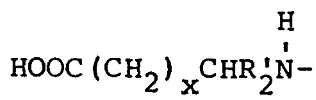

ist;
$A_3$ $-(CH_2)n_3$ oder $-C(CH_2)_3-$ ist und
$n_3$ = 1 bis 5;
m 0 oder 1 ist;
(d)

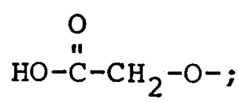

und Y H, Cl oder $OCH_3$ ist;
Z H oder Cl ist;
mit der Maßgabe, daß
(i) wenn W

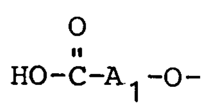

ist, V

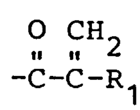

ist und
Y und Z Cl sind;

(ii) wenn W

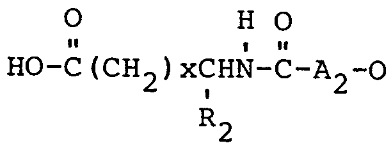

ist, V

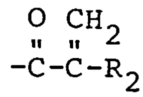

ist,
Y und Z Cl sind, mit der Maßgabe, daß wenn
$R_2$ $C_2H_5$ ist, $R_2'$ H ist und X O ist,
$n_2$ 2 bis 5 ist; mit der Maßgabe, daß wenn
$R_2$ Isopropyi ist, $R_2'$ $CH_3$ ist und X O ist,
$n_2$ 2 bis 5 ist;
(iii) wenn W

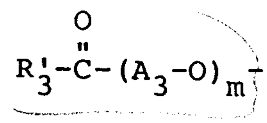

ist, V

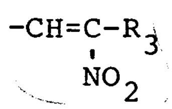

ist;
mit der Maßgabe, daß wenn $R_3'$ OH ist, Y H oder Cl ist und m 1 ist, $n_3$ = 4 oder 5;
(iv) wenn

W 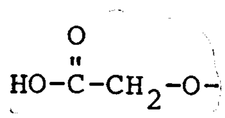

ist, V

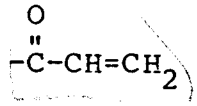

ist;
und Y und Z Cl sind.

**2.** Verbindung und pharmazeutisch annehmbare Salze nach Anspruch 1, ausgewählt aus der Gruppe, welche besteht aus:
a. 3-[2,3-Dichlor-4-(2-methylenbutyryl)phenoxy]propionsäure,

b. 5-[2,3-Dichlor-4-(2-methylenbutyryl)phenoxy]valeriansäure,
c. 6-[2,3-Dichlor-4-(2-methylenbutyryl)phenoxy]hexansäure,
d. 1-[2,3-Dichlor-4-(2-methylenbutyryl)phenoxy]cyclobutan-1-carbonsäure,
e. 1-[2,3-Dichlor-4-(2-methylenvaleryl)phenoxy]cyclobutan-1-carbonsäure.

**3.** Verbindung und pharmazeutisch annehmbare Salze nach Anspruch 1, ausgewählt aus der Gruppe, welche besteht aus:
a. N-{[2,3-Dichlor-4-(2-methylenbutyryl)phenoxy]acetyl}S(+)-alanin,
b. N-{[2,3,-Dichlor-4-(2-methylenbutyryl)phenoxy]acety}S(+)-serin,
c. N-{1-[2,3-Dichlor-4-(2-methylenbutyryl)phenoxy]cyclobutan-1-carbonyl}glycin,
d. N-{1-[2,3-Dichlor-4-(2-methylenvaleryl)phenoxy]cyclobutan-1-carbonyl}glycin,
e. N-{[2,3-Dichlor-4-(2-methylenbutyryl)phenoxy]acetyl}S(-)-phenylalanin,
f. N-{4-[2,3-Dichlor-4-(2-methylenbutyryl)phenoxy]butyryl}glycin,
g. N-{3-[2,3-Dichlor-4-(2-methylenbutyryl)phenoxy]propionyl}glycin,
h. N-{1-[2,3-Dichlor-4-(2-methylenbutyryl)phenoxy]cyclobutan-1-carbonyl}-3-aminopropionsäure,
i. N-[(2,3-Dichlor-4-acryloylphenoxy)acetyl]glycin,
j. N-{1-[2,3-Dichlor-4-(2-methylenbutyryl)phenoxy]cyclobutan-1-carbonyl}S(+)-alanin,
k. N-{[2,3-Dichlor-4-(2-methylenbutyryl)phenoxy]acetyl}S(+)-leucin.

**4.** Verbindung und pharmazeutisch annehmbare Salze nach Anspruch 1, ausgewählt aus der aus den folgenden Verbindungen bestehenden Gruppe, worin:
a. $R_3'$ OH ist, $A_3$ $(CH_2)_4$ ist, $R_3$ $C_2H_5$ ist, Y und Z Cl sind und m 1 ist,
b. $R_3'$ OH ist, $A_3$ $(CH_2)_5$ ist, $R_3$ $C_2H_5$ ist, Y und Z Cl sind und m 1 ist,
c. $R_3'$ OH ist, $A_3$

$$-\dot{C}(CH_2)_3$$

ist, $R_3$ $C_2H_5$ ist, Y und Z Cl sind und m 1 ist,
d. $R_3'$ OH ist, $A_3$ $-C(CH_2)_3$ ist, $R_3$ $CH_2CH_2CH_3$ ist, Y und Z Cl sind, m 1 ist,
e. $R_3'$ $HOOCCH_2NH$ ist, $A_3$ $-(CH_2)_3$- ist, $R_3$ $C_2H_5$ ist, Y und Z Cl sind und m 1 ist,
f. $R_3'$ S(+)-$HOOCCH(CH_3)NH$- ist, $A_3$ $(CH_2)_3$ ist, $R_3$ $C_2H_5$ ist, Y und Z Cl sind und m 1 ist,
g. $R_3'$ S(+)-$HOOCCH[CH_2CH(CH_3)_2]NH$ ist, $A_3$ $(CH_2)_3$ ist, $R_3$ $C_2H_5$ ist, Y und Z Cl sind und m 1 ist,
h. $R_3'$ S(+)-$HOOCCH(CH_2OH)NH$- ist, $A_3$ $(CH_2)_3$ ist, $R_3$ $C_2H_5$ ist, Y und Z Cl sind und m 1 ist,
i. $R_3'$ S(-)-$HOOCCH(CH_2C_6H_5)NH$- ist, $A_3$ $(CH_2)_3$ ist, $R_3$ $C_2H_5$ ist, Y und Z Cl sind und m 1 ist,
j. $R_3'$ $HOOCCH_2NH$- ist, $A_3$ $(CH_2)_3$ ist, $R_3$ $CH_2CH_2CH_3$ ist, Y und Z Cl sind und m 1 ist,
k. $R_3'$ $HOOCCH_2NH$- ist, $A_3$ $-C(CH_2)_3$ ist, $R_3$ $C_2H_5$ ist, Y und Z Cl sind und m 1 ist,
l. $R_3'$ $HOOCCH_2NH$ ist, $A_3$ $(CH_2)_2$ ist, $R_3$ $C_2H_5$ ist, Y und Z Cl sind und m 1 ist,
m. $R_3'$ $HOOCCH_2NH$- ist, $A_3$ $(CH_2)_4$ ist, $R_3$ $C_2H_5$ ist, Y und Z Cl sind und m 1 ist,
n. $R_3'$ $HOOCCH_2NH$- ist, $A_3$ $(CH_2)_3$ ist, $R_3$ $C_2H_5$ ist, Y und Z Cl sind und m 1 ist,
o. $R_3'$ $HOOCCH_2CH_2NH$ ist, $A_3$ $-C(CH_2)_3$ ist, $R_3$ $C_2H_5$ ist, Y und Z Cl sind und m 1 ist,
p. $R_3'$ OH ist, Y $OCH_3$ ist, Z H ist, $R_3$ $CH_3$ ist, $A_3$ $CH_2$ ist und m 1 ist,
q. $R_3'$ OH ist, Y H ist, Z Cl ist, $R_3$ $C_2H_5$ ist und m O ist.

**5.** Verwendung eines Antisichelmittels zur Herstellung eines zur Behandlung von Sichelzellenanämie geeigneten Medikaments, worin das Antisichelmittel eine aus den folgenden allgemeinen Formeln ausgewählte Verbindung ist sowie pharmazeutisch annehmbare Salze davon:
a.

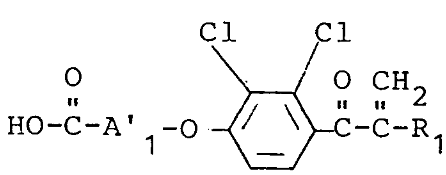

worin $R_1$ Ethyl, Propyl oder Isopropyl ist und

$A_1'$ $-(CH_2)n'_1$, $-C(CH_3)_2-$ oder

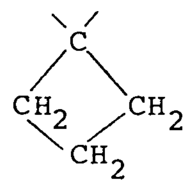

ist und $n_1'$ = 2 bis 5; oder

b.

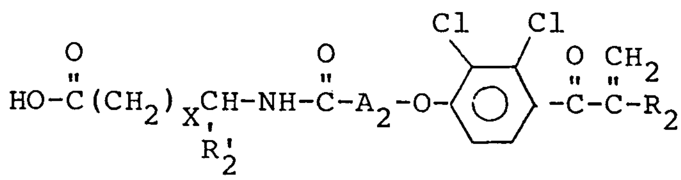

worin

| | |
|---|---|
| $R_2$ = | H oder Niederalkyl; |
| $R_2'$ = | H, Niederalkyl, Benzyl, $CH_2OH$; |
| $A_2$ = | $(CH_2)n_2$, $-C(CH_3)_2$ oder $C(CH_2)_3$; |
| $n_2$ = | 1 bis 5; und |
| x = | 0 oder 1; oder |

c.

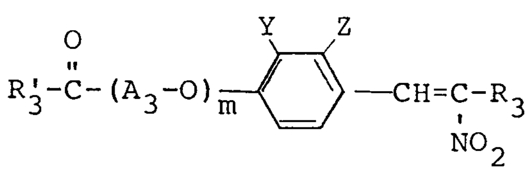

worin

| | |
|---|---|
| $R_3$ = | Niederalkyl; |
| $R_3'$ = | HO oder $HOOC(CH_2)_xCHR_2'$ NH-; |
| $R_2'$ = | H, Niederalkyl, Benzyl oder $-CH_2OH$; |
| x = | 0 oder 1; |
| $A_3$ = | $-(CH_2)n_3-$ oder $-C(CH_2)_3$; |
| $n_3$ = | 1 bis 5; und |
| Y = | H, Cl oder $OCH_3$, |
| Z = | H oder Cl, |
| m = | 0 oder 1; |

d.

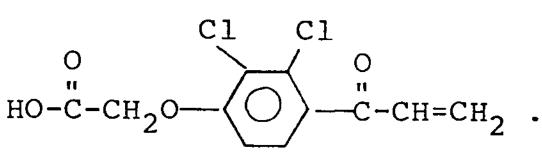

**6.** Pharmazeutische Antisichelzusammensetzung, welche einen pharmazeutischen Träger und eine nicht toxische therapeutisch wirksame Menge eines Antisichelmittels umfaßt, worin das Antisichelmittel ausgewählt ist aus:

a. 3-[2,3-Dichlor-4-(2-methylenbutyryl)phenoxy]propionsäure,

b. 4-[2,3-Dichlor-4-(2-methylenbutyryl)phenoxy]buttersäure,

c. 5-[2,3-Dichlor-4-(2-methylenbutyryl)phenoxy]valeriansäure,
d. 6-[2,3-Dichlor-4-(2-methylenbutyryl)phenoxy]hexansäure,
e. 2-[2,3-Dichlor-4-(2-methylenbutyryl)phenoxy]-2-methylpropionsäure,
f. 1-[2,3-Dichlor-4-(2-methylenbutyryl)phenoxy]cyclobutan-1-carbonsäure,
g. 1-[2,3-Dichlor-4-(2-methylenvaleryl)phenoxy]cyclobutan1-carbonsäure,
h. 4-[2,3-Dichlor-4-(2-methylenvaleryl)phenoxy]buttersäure.

**7.** Pharmazeutische Antisichelzusammensetzung, welche einen pharmazeutischen Träger und eine nicht toxische therapeutisch wirksame Menge eines Antisichelmittels umfaßt, worin das Antisichelmittel ausgewählt ist aus:
a. N-{[[2,3-Dichlor-4-(2-methylenbutyryl)phenoxy]acetyl}-S(+)-alanin,
b. N-{[2,3-Dichlor-4-(2-methylenbutyryl)phenoxy]acetyl}-S(+)-serin,
c. N-{1-[2,3-Dichlor-4-(2-methylenbutyryl)phenoxy]cyclobutan-1-carbonyl}glycin,
d. N-{1-[2,3-Dichlor-4-(2-methylenvaleryl)phenoxy]cyclobutan-1-carbonyl}glycin,
e. N-{[2,3-Dichlor-4-(2-methylenbutyryl)phenoxy]acetyl}-S(-)-phenylalanin,
f. N-{4-[2,3-Dichlor-4-(2-methylenbutyryl)phenoxy]butyryl}glycin,
g. N-{3-[2,3-Dichlor-4-(2-methylenbutyryl)phenoxy]propionyl}glycin,
h. N-{1-[2,3-Dichlor-4-(2-methylenbutyryl)phenoxy]cyclobutan-1-carbonyl}-3-aminopropionsäure,
i. N-[(2,3-Dichlor-4-acryloylphenoxy)acetyl]glycin,
j. N-{1-[2,3-Dichlor-4-(2-methylenbutyryl)phenoxy]cyclobutan-1-carbonyl}-S(+)-alanin,
k. N-{[2,3-Dichlor-4-(2-methylenbutyryl)phenoxy]acetyl}-S(+)-leucin.

**8.** Pharmazeutische Antisichelzusammensetzung, welche einen pharmazeutischen Träger und eine nicht toxische therapeutisch wirksame Menge eines Antisichelmittels umfaßt, worin das Antisichelmittel die Formel c. in Anspruch 5 hat, worin
a. $R_3'$ OH ist, Y und Z H sind, $A_3$ $CH_2$ ist, $R_3$ $CH_3$ ist und m 1 ist,
b. $R_3'$ OH ist, Y und Z Cl sind, $A_3$ $(CH_2)_3$ ist, $R_3$ $C_2H_5$ ist und m 1 ist.